# FASCICULE DE BREVET EUROPEEN

(11) **EP 1 315 708 B1**
(45) Date de publication et mention de la délivrance du brevet: **11.08.2004**
(21) Numéro de dépôt: 01963120.9
(22) Date de dépôt: 23.08.2001
(51) Int. Cl.: C07D 307/80, A61K 31/343, A61K 31/4525, A61P 9/06, A61P 7/04

(54) **AMINOALKENYLBENZOYL-BENZOFURANES OU BENZOTHIOPHENES, LEUR PROCEDE DE PREPARATION ET LES COMPOSITIONS LES CONTENANT**
AMINOALKENYLBENZOYL-BENZOFURANE ODER BENZOTHIOPHENE, VERFAHREN ZUR IHRER HERSTELLUNG UND SIE ENTHALTENDE ZUBEREITUNGEN
AMINOALKENYLBENZOYL-BENZOFURAN OR BENZOTHIOPHENE DERIVATIVES, METHOD FOR PREPARING SAME AND COMPOSITIONS CONTAINING SAME

(30) Priorité: 23.08.2000 FR 0010835
(43) Date de publication de la demande: 04.06.2003
(73) Titulaire: SANOFI-SYNTHELABO, 75013 Paris (FR)
(72) Inventeur: ASSENS, Jean-Louis, F-34790 Grabels (FR); BERNHART, Claude, 34980 Saint-Gely-du-Fesc (FR); CABANEL-HAUDRICOURT, Frédérique, F-34570 Pignan (FR); DOS SANTOS, Victor, F-34130 Valergues (FR); GAUTIER, Patrick, F-34660 Cournonterral (FR); NISATO, Dino, F-34680 Saint Georges d'Orques (FR)
(74) Mandataire: Weber, Mathieu
(86) Numéro de dépôt international: PCT/FR2001/002656
(87) Numéro de publication internationale: WO 2002/016338

(56) Documents cités:
- WO-A-96/28152
- WO-A-97/01549
- WO-A-99/54325
- US-A- 5 622 974
- US-A- 5 827 876

## Description

La présente invention se rapporte, d'une manière générale, à de nouveaux dérivés hétérocycliques ainsi qu'à leur procédé de préparation.

Plus précisément, l'invention concerne de nouveaux dérivés de benzofurane ou de benzothiophène, lesquels peuvent être représentés par la formule générale : 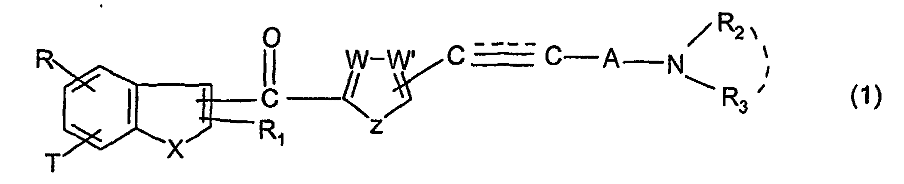 ainsi que leurs sels pharmaceutiquement acceptables, dans laquelle :
C 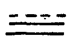 C représente le groupement -CH=CH- ou le groupement -C≡C-
A représente un groupement alkylène, linéaire ou ramifié, en C₁-C₃ ou alkénylène en C₂-C₃,
T représente l'hydrogène ou un radical alkyle en C₁-C₄,
R représente :
   - le groupement cyano, hydroxyméthyle, formyle ou tétrazolyle,
   - un groupement ester de formule générale: 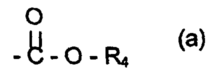 dans laquelle R₄ représente un groupement alkyle en C₁-C₆ ou cycloalkyle en C₃-C₆,
   - un groupement carboxyle de formule générale : 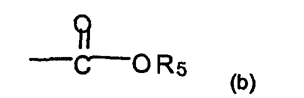 dans laquelle R₅ représente l'hydrogène ou un atome de métal alcalin,
   - un groupement amide de formule générale : 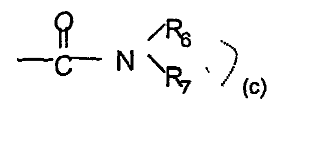 dans laquelle R₆ et R₇, identiques ou différents, représentent l'hydrogène ou un radical alkyle, linéaire ou ramifié, en C₁-C₄ ou R₆ et R₇ , lorsqu'ils sont pris ensemble, représentent une chaîne alkylène en C₂-C₆,
   - un groupement de formule générale : 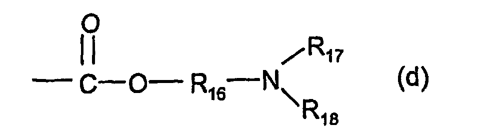 dans laquelle R₁₆ , R₁₇ et R₁₈ , identiques ou différents, représentent un groupement alkylène, linéaire ou ramifié, en C₁-C₄,
R₁ représente l'hydrogène, un groupement alkyle, linéaire ou ramifié, en C₁-C₆ , cycloalkyle en C₃-C₆, ou phényle,
R₂ et R₃, identiques ou différents, représentent l'hydrogène, un groupement alkyle, linéaire ou ramifié, en C₁-C₆ ou un groupement cycloalkyle en C₃-C₆,
ou R₂ et R₃, lorsqu'ils sont pris ensemble, représentent un groupement alkylène en C₃-C₁₀, linéaire ou ramifié,
ces alternatives R₂ et R₃ identiques ou différents et R₂ et R₃ pris ensemble étant représentées dans la formule (1) par le symbole 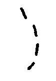 situé entre R₂ et R₃
W, W' et Z sont tels que :
   - lorsque W et W', identiques représentent CH, Z représente -O- ou -S-
   - lorsque W représente CH et W' représente C-R₈, Z représente 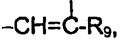 R₈ et R₉ étant identiques ou différents et représentant l'hydrogène, un atome d'halogène par exemple chlore ou brome, un radical alkyle en C₁-C₄ tel que méthyle ou un radical alkoxy en C₁-C₄ tel que méthoxy,
X représente -O- ou -S-,
ces dérivés de benzofurane ou de benzothiphène étant sous formes d'isomères individuels ou de mélanges de ceux-ci.

En particulier, les dérivés de benzofurane ou de benzothiopène selon l'invention sont caractérisés en ce que
C 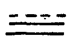 C représente le groupement -CH=CH-

Des classes de composés préférés de l'invention peuvent être représentées par les composés de formule (1) :
- dans laquelle R représente un groupement isopropoxycarbonyle
- ou dans laquelle R₁ et/ou R₂ et/ou R₃ représentent le groupement n-butyle
- ou dans laquelle X représente -O-.
Une autre classe de composés préférés de formule (1) est celle dans laquelle 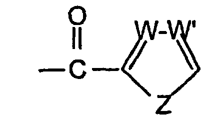 représente le radical benzoyle.

De même, une classe particulière de composés de formule (1) est celle dans laquelle l'entité : 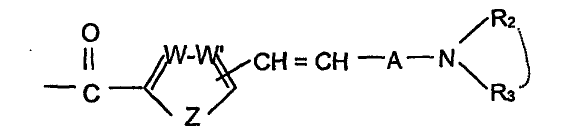 représente un radical benzoyle substitué en position 4 par un groupement

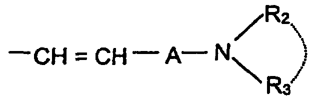

Enfin, les composés de formule (1) dans laquelle R₁ représente n-butyle, A représente le groupement méthylène et R₂ et R₃, identiques, représentent le groupement n-butyle peuvent être considérés comme préférés.

Les composés de formule (1) peuvent se présenter sous forme d'isomères géométriques E ou Z.

En conséquence, l'invention se rapporte à la fois aux isomères individuels des composés de formule (1) ainsi qu'à leurs mélanges.

En outre, les isomères E des composés de formule (1) constituent des composés préférés.

L'invention se rapporte également aux sels pharmaceutiquement acceptables des composés de formule (1) formés à partir d'un acide organique ou inorganique.

Comme exemples de sels organiques de ce genre, on peut citer les oxalate, maléate, fumarate, méthanésulfonate, benzoate, ascobate, pamoate, succinate, hexamate, bisméthylènesalicylate, éthanedisulfonate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophylline acétate ainsi que les sels formés à partir d'un acide aminé tel que le sel de lysine ou l'histidine.

Comme sels inorganiques de ce genre, on peut citer les chlorhydrate, bromhydrate, sulfate, sulfamate, phosphate et nitrate.

En particulier, on peut citer le chlorhydrate de 2-butyl-3-[4-[(E)-3-(dibutylamino)-1-propenyl]benzoyl]-1-benzofurane-5-carboxylate d'isopropyle.

On a trouvé que les composés de l'invention possèdent de remarquables propriétés pharmacologiques notamment des propriétés anti-arythmiques puisqu'ils se sont révélés capables de supprimer ou de prévenir les troubles du rythme ventriculaire et auriculaire. La plupart des composés de l'invention ont des propriétés électrophysiologiques des classes 1, 2, 3 et 4 de la classification de Vaughan-Williams qui confèrent des propriétés bradycardisantes, anti-hypertensives et antiadrénergiques α et β non compétitives. De plus, la plupart des composés ont également révélé des propriétés anti-oxydantes, une affinité pour les récepteurs sigma et une capacité à augmenter la synthèse du NO.

Par ailleurs, ces composés de l'invention manifestent des propriétés inhibitrices de différents agents hormonaux tels que par exemple l'angiotensine II, l'arginine vasopressine, le neuropeptide Y ou l'endothéline.

Ces propriétés sont capables de rendre les composés en question très utiles dans le traitement de certains syndromes pathologiques du système cardio-vasculaire en particulier dans le traitement de l'angine de poitrine, de l'hypertension, de l'arythmie en particulier atriale, ventriculaire ou supraventriculaire, de l'insuffisance circulatoire cérébrale. De même, les composés de l'invention pourront être utilisés dans le traitement de l'insuffisance cardiaque, de l'infarctus du myocarde compliqué ou non d'insuffisance cardiaque ou pour la prévention de la mortalité post-infarctus.

Dans le domaine antitumoral, les composés de l'invention pourront être utiles comme potentialisateurs d'anticancéreux.

En conséquence, l'invention se rapporte également à un médicament, caractérisé en ce qu'il comprend un composé dérivé de benzofurane ou de benzothiophène, ou un sel pharmaceutiquement acceptable de ce dernier, selon l'invention.

En conséquence, l'invention se rapporte également à des compositions pharmaceutiques ou vétérinaires contenant comme principe actif, au moins un composé de l'invention, en association avec un véhicule pharmaceutique ou excipient approprié.

Selon la voie d'administration choisie, la posologie journalière pour un être humain pesant 60 kg se situera entre 2 et 2000mg de principe actif, en particulier entre 50 et 500 mg de principe actif.

Les composés de formule (1) peuvent être préparés selon les méthodes suivantes :
**A.** - Dans le cas où R représente le groupement cyano, le groupement formyle, un groupement (a) ou un groupement (c) et lorsque le composé de formule (1) est sous forme d'isomère de configuration E, en faisant réagir un composé de formule générale : 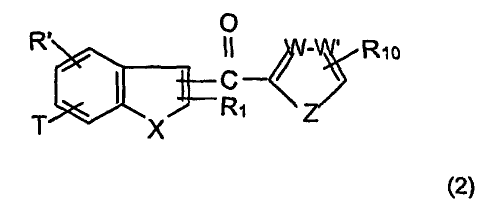 dans laquelle R' représente le groupement cyano, le groupement formyle, un groupement (a) ou un groupement (c), R₁₀ représente un atome d'halogène de préférence brome ou iode ou le groupement trifluorométhanesulfonyloxy et R₁, T, X, W, W' et Z ont la même signification que précédemment, avec un dérivé organostannique de configuration E répondant à la formule générale : 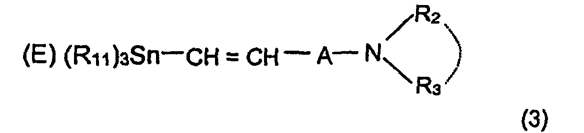 dans laquelle A, R₂ et R₃ ont la même signification que précédemment et R₁₁ représente un radical alkyle en C₁-C₄ en particulier butyle, après protection de la fonction amine lorsque R₂ et/ou R₃ représentent l'hydrogène et ce, en présence de chlorure de lithium et d'un dérivé organopalladié tel que le tétrakis (triphénylphosphine)palladium, puis en déprotégeant, si nécessaire, le composé ainsi formé, ce qui fournit, sous forme de base libre, les composés désirés de formule (1).
   Habituellement la réaction se déroule à la température de reflux du solvant qui peut être par exemple un éther tel que le dioxane.
   En outre, la protection de la fonction amine du composé de formule (3), c'est-à-dire la protection envisagée lorsque R₂ et/ou R₃ représentent l'hydrogène peut être obtenue par exemple par traitement au moyen d'un composé permettant la fixation d'un groupement aisément éliminable notamment au moyen d'un anhydride de t-butoxycarbonyle et la déprotection s'opère par la suite, dans ce cas, après traitement en milieu acide.
**B.** - Dans le cas où R représente le groupement cyano, le groupement formyle, un groupement (a) ou un groupement (c) et lorsque le composé de formule (1) est sous forme d'isomère de configuration Z, en hydrogénant un composé alkynyle de formule générale : 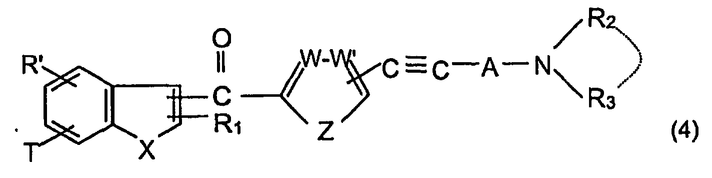 dans laquelle A, R', R₁, R₂, R₃, T, W, W', X et Z ont la même signification que précédemment et ce, en présence d'un catalyseur approprié tel que le charbon palladié ce qui fournit, sous forme de base libre, les composés désirés de formule (1).
   Habituellement, la réaction s'effectue dans un solvant tel qu'un hydrocarbure aromatique par exemple le toluène et à la température ambiante.
**C.** - Dans le cas où R représente un groupement (b), en saponifiant en présence d'un agent basique à savoir un hydroxyde de métal alcalin, par exemple l'hydroxyde de sodium, un composé de formule (1) ci-dessus dans laquelle R représente un groupement (a), c'est-à-dire un composé de formule générale : 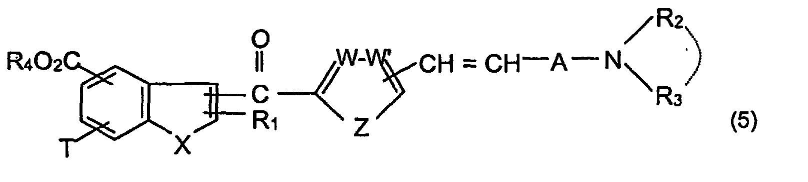 dans laquelle A, R₁, R₂, R₃, R₄, T, W, W' et Z ont la même signification que précédemment, ce qui fournit, sous forme de base libre, les composés de formule (1) dans laquelle R₅ représente un atome de métal alcalin, composés que l'on traite, si nécessaire, avec un acide fort, par exemple l'acide chlorhydrique, ce qui fournit, sous forme de base libre, les composés désirés de formule (1) dans laquelle R₅ représente l'hydrogène.
**D.** - Dans le cas où R représente le groupement hydroxyméthyle, en déprotégeant un cétal de formule générale : 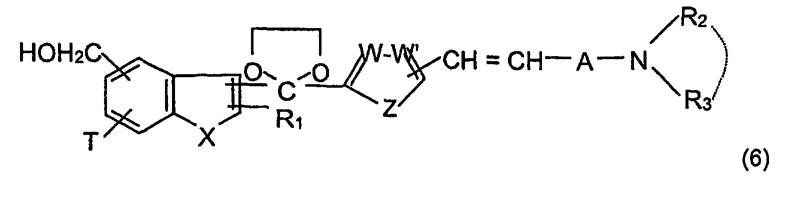 dans laquelle A, R₁, R₂, R₃, T, X, W, W' et Z ont la même signification que précédemment et ce, au moyen de pyridine p-toluènesulfonate et de préférence à la température de reflux, ce qui fournit les composés désirés de formule (1) sous forme de base libre.
   Les dérives benzofuraniques ou benzothiophéniques de formule (1), qui répondent également à la formule générale : 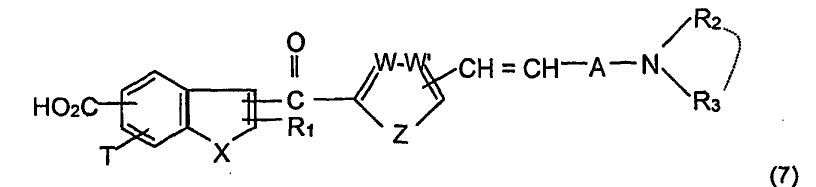 dans laquelle A, R₁, R₂, R₃, T, W, W', X et Z ont la même signification que précédemment, sont eux-mêmes intermédiaires de synthèse pour la préparation de composés de formule (1 ).
**E** - Par exemple, on peut préparer, de manière alternative, les composés de formule (1) dans laquelle R représente un groupement (c) dans lequel R₆ et R₇ sont identiques et représentent chacun l'hydrogène, en faisant réagir un composé de formule (7) en question au moyen de dicyclohexylcarbodiimide en présence d'hydroxybenzotriazole et d'ammoniaque, ce qui fournit les composés désirés de formule (1) sous forme de base libre.
   D'autres composés de formule (1) peuvent être utilisés comme intermédiaires de synthèse de composés de l'invention notamment les dérivés cyano qui répondent également à la formule générale : 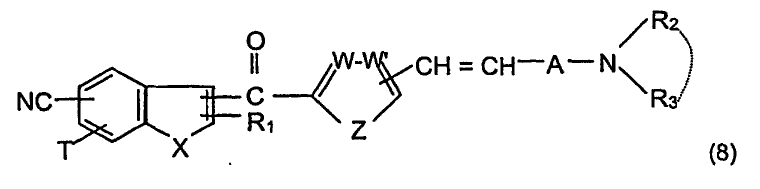 dans laquelle A, R₁, R₂, R₃, T, W, W', X et Z ont la même signification que précédemment.
   Ainsi, on peut mettre en oeuvre les méthodes suivantes, c'est-à-dire :
**F.** - Dans le cas où R représente un groupement (c) dans lequel R₆ et R₇ représentent chacun l'hydrogène, on hydrolyse un composé de formule (8) en présence d'un acide fort tel que par exemple l'acide sulfurique et généralement à la température ambiante, ce qui fournit, sous forme de base libre, les composés désirés de formule (1).
**G.** - Dans le cas où R représente le groupement tétrazolyle, on fait réagir de préférence dans un solvant aprotique tel qu'un hydrocarbure aromatique, par exemple le benzène ou le toluène et habituellement à la température de reflux du milieu, un composé de formule (8) avec un (trialkyl en C₁-C₄) azido étain par exemple l'azide de tributylétain, ce qui fournit, sous forme de base libre, les composés désirés de formule (1).
   Les méthodes décrites précédemment permettent d'obtenir les composés de formule (1) sous forme d'isomères individuels E ou Z ou sous forme de mélanges de ceux-ci selon le procédé mis en oeuvre.
   Si nécessaire, ces isomères peuvent être produits sous forme séparée à partir de leurs mélanges par mise en oeuvre de méthodes connues telles que par exemple chromatographie ou précipitation.
**H.** - Les composés de formule (1) obtenus sous forme de base libre selon l'une ou l'autre des méthodes décrites ci-dessus, peuvent ensuite être transformés, si nécessaire, en sels pharmaceutiquement acceptables par réaction avec un acide organique ou inorganique approprié par exemple l'acide oxalique, maléique, fumarique, méthanesulfonique, benzoïque, ascorbique, pamoïque, succinique, hexamique, bisméthylènesalicylique, éthanedisulfonique, acétique, propionique, tartrique, salicylique, citrique, gluconique, lactique, malique, cinnamique, mandélique, citraconique, aspartique, palmitique, stéarique, itaconique, glycolique, p-aminobenzoïque, glutamique, benzènesulfonique, p-toluènesulfonique, théophylline acétique, la lysine ou l'histidine ou encore l'acide chlorhydrique, bromhydrique, sulfurique, sulfamique, phosphorique ou nitrique.

Les composés de formule (2) peuvent être obtenus :
a) lorsque R₁₀ représente un atome d'halogène, en faisant réagir un composé de formule générale : 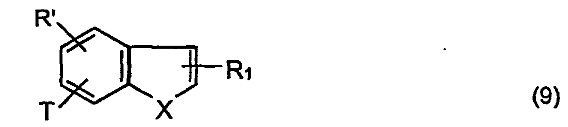 dans laquelle R', R₁ et T ont la même signification que précédemment, avec un halogénure de formule générale: 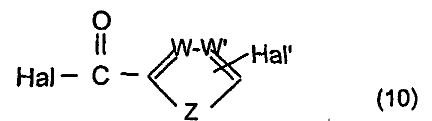 dans laquelle W, W' et Z ont la même signification que précédemment et Hal et Hal', identiques ou différents, représentent un atome d'halogène de préférence brome ou iode et ce, en présence d'un acide de Lewis tel que le chlorure d'aluminium, le chlorure ferrique ou le chlorure stannique, pour former les composés désirés de formule (2).
b) lorsque R₁₀ représente un groupement trifluorométhanesulfonyloxy, en faisant réagir un composé de formule générale : 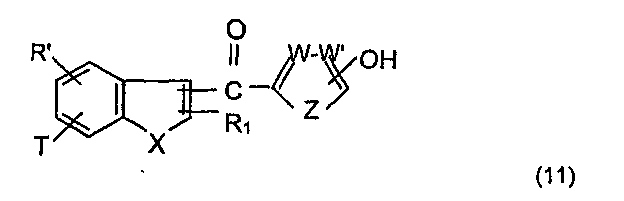 dans laquelle R', R₁, T, W, W', X et Z ont la même signification que précédemment et ce, avec l'anhydride trifluorométhanesulfonique en présence d'un accepteur d'acide tel que la pyridine, ce qui fournit les composés désirés de formule (2).

En ce qui concerne les composés stanniques de formule (3), on peut les obtenir en faisant réagir une alkynylamine de formule générale : 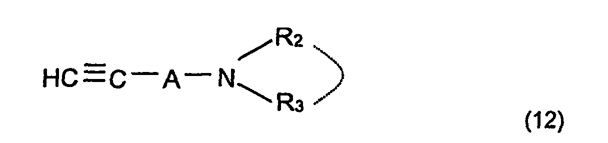 dans laquelle A, R₂ et R₃ ont la même signification que précédemment, après protection de la fonction amine lorsque R₂ et/ou R₃ représentent l'hydrogène et ce, avec un hydrure de formule générale :

(R₁₁)₃ Sn - H (13)

dans laquelle R₁₁ a la même signification que précédemment, ce qui fournit les composés désirés de formule (3).

Quant aux composés de formule (4), on peut les préparer en faisant réagir un composé de formule (2) avec un dérivé alkyne de formule (12), après protection de la fonction amine lorsque R₂ et/ou R₃ représentent l'hydrogène et ce, en présence d'un composé organopalladié comme catalyseur essentiellement un sel de palladium (II) de préférence complexé avec au moins un composé organophosphoré comprenant du phosphore trivalent par exemple le dichloro bis-(triphénylphosphine)palladium, puis on déprotège, si nécessaire le composé ainsi formé, ce qui fournit les composés désirés de formule (4).

La protection de la fonction amine du composé de formule (12) c'est-à-dire la protection envisagée lorsque R₂ et/ou R₃ représentent l'hydrogène peut être obtenue par exemple par traitement au moyen d'un composé permettant la fixation d'un groupement aisément éliminable notamment au moyen d'un anhydride de t-butyoxycarbonyle et la déprotection s'opère par la suite, dans ce cas, par traitement en milieu acide.

Les composés de formule (6) peuvent être préparés au départ d'un ester de formule (1) dans laquelle R représente un groupement (a) :
(a) en traitant cet ester de formule (1) à la température de reflux du milieu, au moyen de glycol en présence d'acide p-toluènesulfonique, pour former un diéther de formule générale : 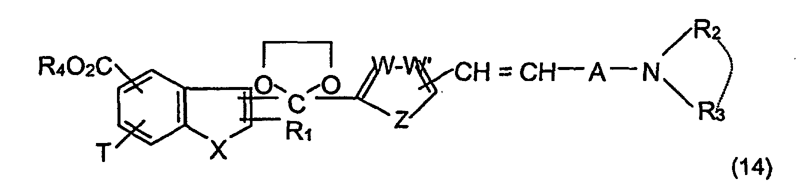 dans laquelle A, R₁, R₂, R₃, R₄, T, X, W, W' et Z ont la même signification que précédemment,
(b) en réduisant ce composé de formule (14) au moyen d'un hydrure de métal alcalin tel que l'hydrure de lithium aluminium et dans un solvant tel qu'un éther pour obtenir les composés désirés.

Les composés de formule (9) dans laquelle R' est situé en position 5 et représente le groupement cyano ou un groupement (a) et R₁ est situé en position 2 peuvent être préparés selon la suite d'étapes ci-après :
a) **soit**, on traite un dérivé cyano de formule générale : 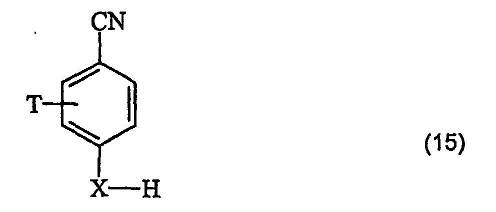 dans laquelle T et X ont la même signification que précédemment, avec l'iode en présence d'ammoniaque, pour former un dérivé iodo de formule générale : 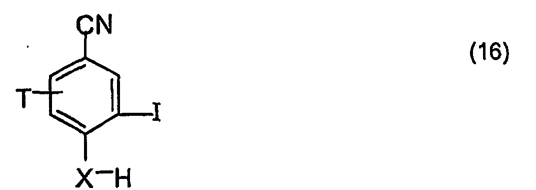 dans laquelle T et X ont la même signification que précédemment,
   **soit**, on traite un dérivé d'acide benzoïque de formule générale : 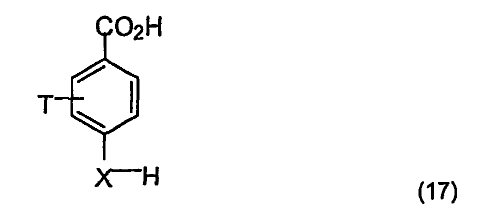 dans laquelle T et X ont la même signification que précédemment, d'abord avec un iodure de métal alcalin et un agent oxydant tel qu'un hypochlorite de métal alcalin, par exemple l'hypochlorite de sodium, ensuite avec un alcool de formule générale :

   R₄-OH (18)

   dans laquelle R₄ a la même signification que précédemment, ce qui fournit un dérivé iodo de formule générale : 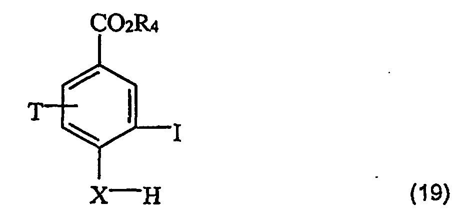 dans laquelle R₄, T et X ont la même signification que précédemment,
b) on fait réagir le dérivé iodé de formule (16) ou (19) avec un dérivé acétylénique de formule générale :

   HC≡C- R₁ (20)

   dans laquelle R₁ a la même signification que précédemment et ce, en présence d'un catalyseur approprié tel qu'un dérivé de palladium, par exemple le tétrakis (triphénylphosphine)palladium et d'iodure cuivreux, ce qui fournit les composés désirés de formule (2).

Si nécessaire, la préparation de l'ester de formule (19) peut être réalisée selon la méthode décrite ci-dessus mais au départ d'un acide de formule (17) sous forme d'halogénure d'acyle obtenu après traitement du dérivé d'acide benzoïque de formule (17) au moyen d'un agent halogénant, par exemple le chlorure de thionyle, le phosgène ou le chlorure d'oxalyle.
**B. -** Les composés de formule (9) dans laquelle R' est situé en position 6 et représente le groupement cyano ou un groupement (a) et R₁ est situé en position 2 peuvent être obtenus comme suit :
   a) on fait réagir un composé de formule générale : 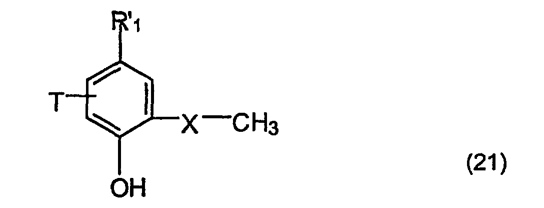 dans laquelle T et X ont la même signification que précédemment et R'₁ représente le groupement cyano ou un groupement (a), avec l'anhydride trifluorométhanesulfonique en présence de pyridine, pour obtenir un composé de formule générale : 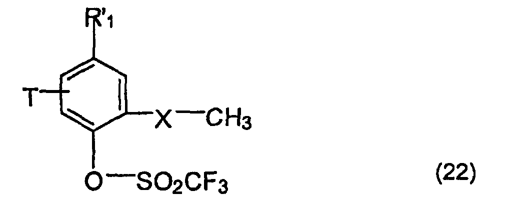 dans laquelle R'₁, T et X ont la même signification que précédemment,
   b) on fait réagir le composé ainsi formé avec un dérivé acétylénique de formule (20) et ce, en présence d'un catalyseur approprié, par exemple un dérivé de palladium tel que le dichloro bis-(triphénylphosphine)palladium et d'un accepteur d'acide tel que la triéthylamine, pour former les composés de formule générale : 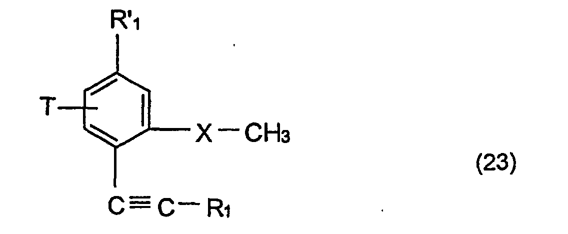 dans laquelle R'₁, R₁, T et X ont la même signification que précédemment,
   c) on cyclise alors ce composé de formule (23) en présence de tribromure de bore à une température inférieure à -50°C, ce qui fournit les composés hétérocycliques de formule générale : 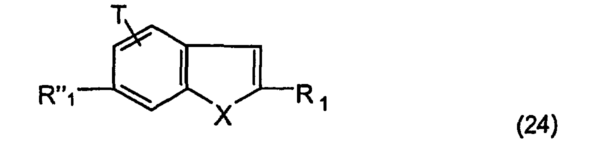 dans laquelle R₁, T et X ont la même signification que précédemment et R"₁ représente le groupement cyano ou carboxylique, ce qui fournit soit des composés désirés de formule (9) lorsque R"₁ représente le groupement cyano soit un acide lorsque R"₁ représente le groupement carboxylique,
   d) on estérifie cet acide avec un alcool de formule (18), ce qui fournit des composés désirés de formule (9).
**C.** - Les composés de formule (9) dans laquelle R' est situé en position 4 et représente le groupement cyano ou un groupement (a) et R₁ est situé en position 2 peuvent être obtenus comme suit :
   a) on fait réagir un composé de formule générale : 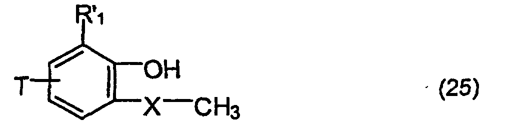 dans laquelle R'₁, T et X ont la même signification que précédemment, avec l'acide trifluorométhanesulfonique en présence de pyridine, pour obtenir un composé de formule générale : 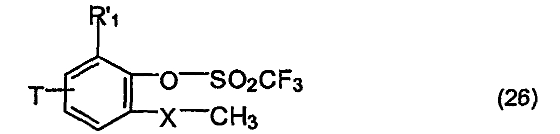 dans laquelle R'₁, T et X ont la même signification que précédemment,
   b) on fait réagir le composé ainsi formé avec un dérivé acétylénique de formule (20) et ce, en présence d'un catalyseur approprié tel qu'un dérivé de palladium par exemple le dichloro bis-(triphénylphosphine)palladium et d'un accepteur d'acide tel que la triéthylamine, pour former les composés de formule générale : 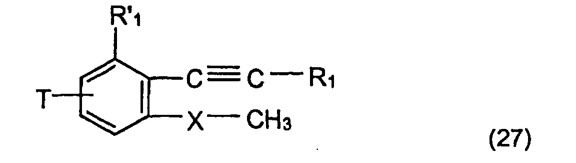 dans laquelle R'₁, R₁ et X ont la même signification que précédemment,
   c) on cyclise alors ce composé de formule (27) en présence de tribromure de bore, ce qui fournit les composés hétérocycliques de formule générale : 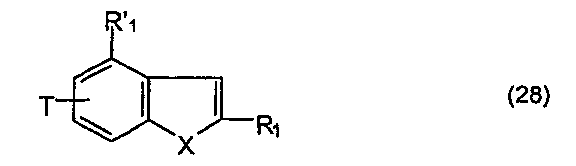 dans laquelle R'₁, R₁, T et X ont la même signification que précédemment, qui correspondent aux composés désirés de formule (9).

De manière alternative, les composés de formule (9) dans laquelle R' est situé en position 5 et représente le groupement cyano ou un groupement (a) et R₁ est en position 2 peuvent également être préparés comme suit :
**I.** Lorsque R' représente un groupement (a) :
   a) on traite d'abord un benzoate de formule générale : 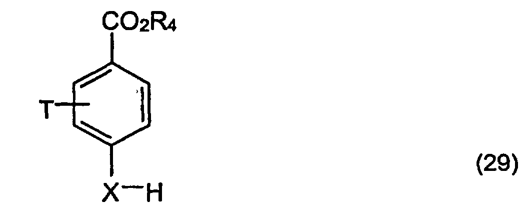 dans laquelle R₄, T et X ont la même signification que précédemment, avec l'acide méthanesulfonique en présence de pentoxyde de phosphore et d'hexaméthylènetétramine, pour donner un dérivé formyle de formule générale : 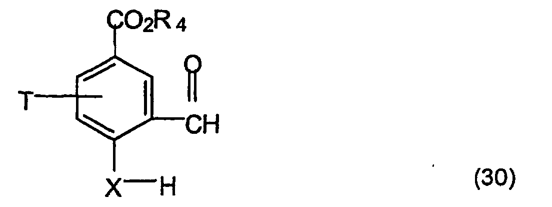 dans laquelle R₄, T et X ont la même signification que précédemment,
   b) on fait ensuite réagir ce composé de formule (30) avec un ester de formule générale ; 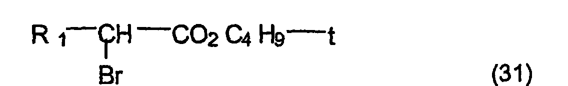 dans laquelle R₁ a la même signification que précédemment, ce qui fournit les composés de formule générale : 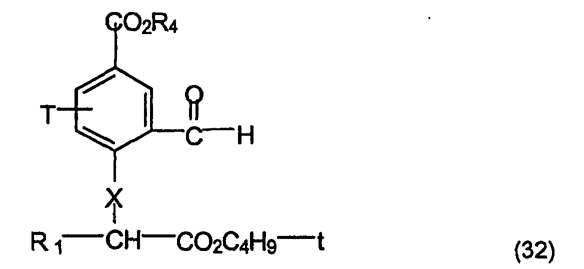 dans laquelle R₁, R₄, T et X ont la même signification que précédemment,
   c) on traite cet ester de formule (32) avec l'acide formique ou l'acide trifluoracétique, ce qui fournit les acides de formule générale : 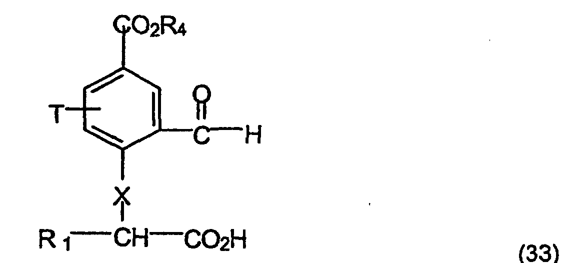 dans laquelle R₁, R₄, T et X ont la même signification que précédemment,
   d) on cyclise ce composé (33) en présence de chlorure de benzènesulfonyle ou de p-toluènesulfonyle et d'un accepteur d'acide tel que la triéthylamine, ce qui donne les composés souhaités de formule (9).
**II.** Lorsque R' représente le groupement cyano :
   a) on traite d'abord un dérivé formyle de formule générale : 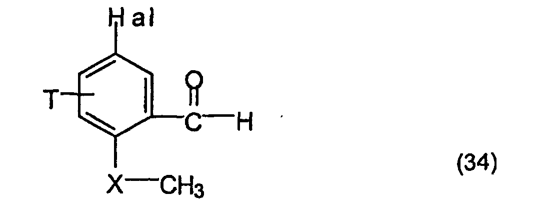 dans laquelle Hal, T et X ont la même signification que précédemment, avec le cyanure de zinc en présence d'un catalyseur approprié, par exemple un dérivé de palladium tel que le tétrakis-(triphénylphosphine)palladium, ce qui fournit les composés de formule générale : 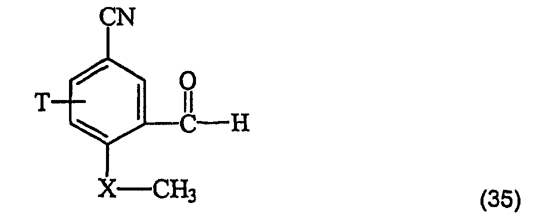 dans laquelle T et X ont la même signification que précédemment,
   b) on déméthyle ensuite ce composé de formule (35) avec le chlorure de lithium, ce qui fournit les composés de formule générale : 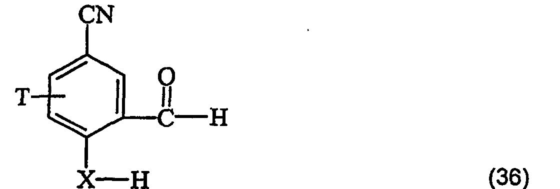 dans laquelle T et X ont a la même signification que précédemment,
   c) on traite alors ce composé de formule (36) avec un ester de formule générale : 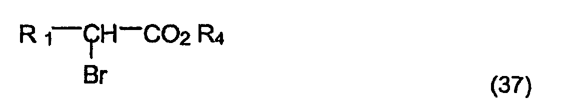 dans laquelle R₁ et R₄ ont la même signification que précédemment et ce, en présence d'un agent basique tel qu'un carbonate de métal alcalin, ce qui donne les composés de formule générale : 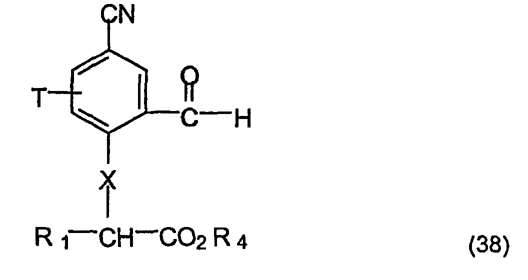 dans laquelle R₁, R₄, T et X ont la même signification que précédemment,
   d) et e) on saponifie cet ester de formule (38) en présence d'un agent basique tel qu'un hydroxyde de métal alcalin et l'on cyclise l'acide ainsi obtenu en présence de chlorure de benzènesulfonyle ou de p-toluènesulfonyle et d'un accepteur d'acide tel que la triéthylamine, ce qui fournit les composés souhaités.

Les composés de formule (9) dans laquelle R' est situé en position 7 et représente le groupement cyano ou un groupement (a) et R₁ est situé en position 2 peuvent être obtenus selon la suite d'étapes ci-après :
a) on traite un alcool de formule générale : 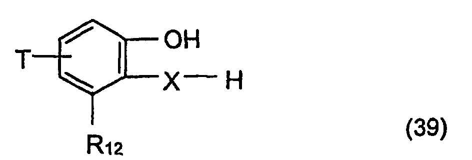 dans laquelle R₁₂ représente un groupement cyano ou formyle et T et X ont la même signification que précédemment et ce, avec l'iodure de méthyle en présence d'un hydrure de métal alcalin pour donner un composé de formule générale : 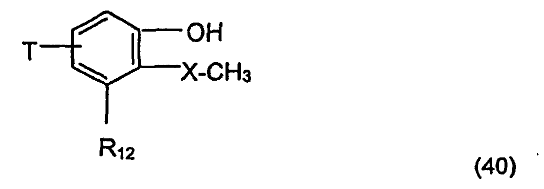 dans laquelle R_{12,} T et X ont la même signification que précédemment,
b) on fait réagir le composé ainsi formé avec l'anhydride trifluorométhanesulfonique pour former un composé de formule générale : 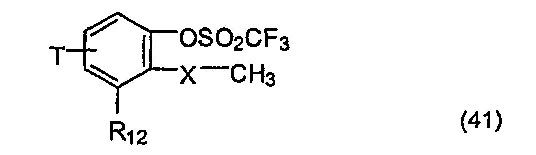 dans laquelle R₁₂, T et X ont la même signification que précédemment,
c) on traite le composé ainsi formé avec un composé de formule (20) en présence d'un catalyseur approprié tel qu'un dérivé de palladium, par exemple le dichloro bis-(triphénylphosphine)palladium, ce qui produit le composé de formule générale : 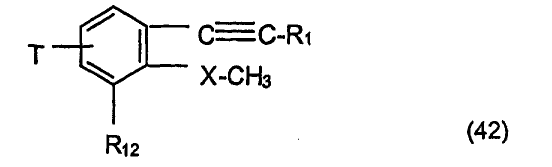 dans laquelle R₁, R₁₂, T et X ont la même signification que précédemment,
d) on fait ensuite réagir le composé de formule (42) ainsi formé :
   - lorsque R₁₂ représente le groupement cyano, avec le chlorure de lithium pour former les composés désirés de formule (9) dans laquelle R' représente le groupement cyano,
   - lorsque R₁₂ représente le groupement formyle, avec un cyanure de métal alcalin en présence d'oxyde manganeux et d'acide acétique pour donner un composé de formule générale : 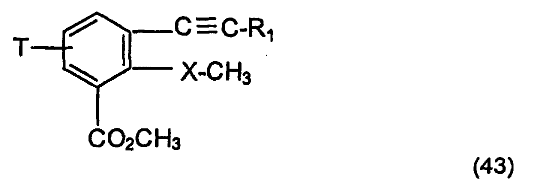
   dans laquelle R₁, T et X ont la même signification que précédemment, que l'on cyclise avec le chlorure de lithium pour donner un mélange d'ester et d'acide de formule générale : 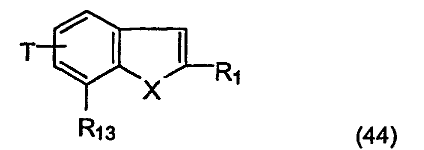 dans laquelle R₁, X et T ont la même signification que précédemment et R₁₃ représente le groupement méthoxycarbonyle ou carboxylique, mélange que l'on traite avec le méthanol en présence d'un acide fort tel que l'acide sulfurique, ce qui fournit les composés désirés de formule (9) dans laquelle R' représente le groupement méthoxycarbonyle.
   Les autres composés de formule (9), c'est-à-dire les composés de formule (9) dans laquelle R' situé en position 7 représente un groupement (a), à l'exception du groupement méthoxycarbonyle, peuvent être obtenus en saponifiant un ester de formule (9) dans laquelle R' situé en position 7 représente le groupement méthoxycarbonyle et ce, en présence d'un agent basique tel qu'un hydroxyde de métal alcalin pour donner un sel que l'on acidifie avec un acide fort tel que l'acide chlorhydrique pour donner un dérivé de 7-carboxy-benzofurane que l'on estérifie avec un alcool de formule générale :

   R'₄ - OH (45)

   dans laquelle R'₄ représente un groupement alkyle en C₂-C₆ ou cycloalkyle en C₃-C₆, ce qui fournit des composés désirés de formule (9).
   Les composés de formule (9) dans laquelle R' représente le groupement formyle peuvent être préparés en oxydant avec le chlorure d'oxalyle, un alcool de formule générale : 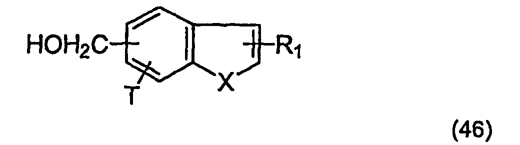 dans laquelle R₁, T et X ont la même signification que précédemment, pour fournir les composés désirés.

De même, les composés de formule (9) dans laquelle R' représente un groupement (c) peuvent être obtenus en :
* saponifiant un ester de formule (9) dans laquelle R' représente un groupement (a) et ce, au moyen d'un hydroxyde de métal alcalin pour obtenir un dérivé de métal alcalin,
* traitant le dérivé de métal alcalin en question au moyen d'un acide fort tel que l'acide chlorydrique pour former un acide,
* halogénant cet acide au moyen d'un agent d'halogénation approprié tel que le chlorure de thionyle pour obtenir un halogénure d'acyle,
* en amidifiant l'halogénure d'acyle ainsi obtenu et ce, au moyen d'un composé de formule générale : 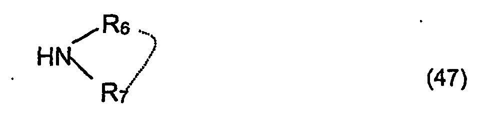
dans laquelle R₆ et R₇ ont la même signification que précédemment, pour former les composés désirés de formule (9).

Les composés de formule (10) peuvent être obtenus moyennant la suite d'étapes ci-après :
a) acylation d'un composé de formule générale : 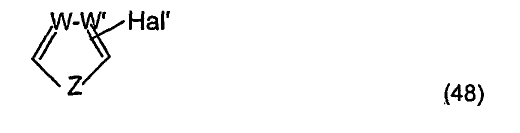 dans laquelle Hal', W, W' et Z ont la même signification que précédemment, avec le chlorure d'acétyle et ce, en présence d'un acide de Lewis tel que le chlorure d'aluminium, pour former un composé de formule générale : 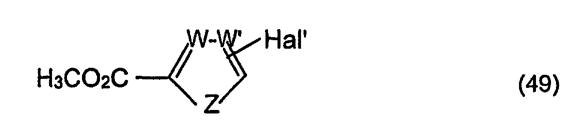 dans laquelle Hal', W, W' et Z ont la même signification que précédemment,
b) réaction du composé ainsi formé d'abord avec le brome en présence d'un hydroxyde de métal alcalin et ensuite avec un acide fort tel que l'acide chlorhydrique ou sulfurique, pour obtenir les acides de formule générale : 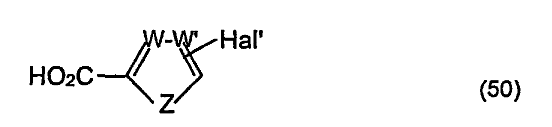 dans laquelle Hal', W, W' et Z ont la même signification que précédemment,
c) halogénation de l'acide ainsi formé par traitement au moyen par exemple de chlorure de thionyle, pour obtenir les composés désirés de formule (10).

En ce qui concerne les composés de formule (11) on peut les obtenir en faisant réagir un dérivé benzofuranique ou benzothiophénique de formule (9) un halogénure de formule générale : 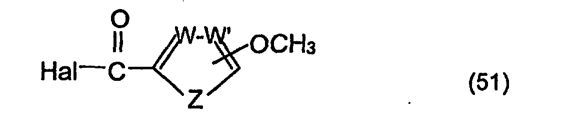 dans laquellè Hal représente un atome d'halogène tel que chlore ou brome et ce, en présence d'un acide de Lewis comme catalyseur par exemple le chlorure ferrique, le chlorure d'aluminium ou le tétrachlorure d'étain, ce qui fournit les dérivés méthoxy de formule générale : 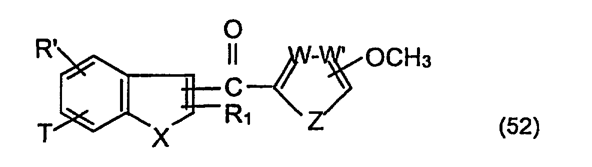 dans laquelle R', R₁, T, W, W', X et Z ont la même signification que précédemment, dérivés méthoxy que l'on déméthyle par chauffage en présence par exemple de chlorure d'aluminium, pour former les composés désirés.

D'une autre manière, on peut obtenir les composés de formule (11) ou de formule (52) dans lesquelles R' représente un groupement (a), un groupement (b) ou un groupement (c) pour mise en oeuvre de différentes méthodes selon lesquelles :
* on saponifie un ester de formule générale : 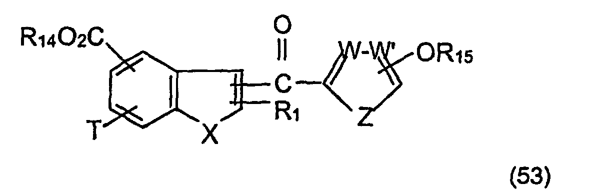 dans laquelle R₁, T, W, W', X et Z ont la même signification que précédemment R₁₄ représente un radical alkyle en C₁-C₄, de préférence méthyle ou éthyle et R₁₅ représente l'hydrogène ou le radical méthyle et ce, avec un hydroxyde de métal alcalin pour obtenir les composés de formule (11) ou de formule (52) dans lesquelles R' représente un groupement (b) dans lequel R₆ représente un atome de métal alcalin,
* on traite le dérivé de métal alcalin ainsi formé avec un acide fort tel que l'acide chlorhydrique pour obtenir un acide c'est-à-dire les composés de formule (11) ou de formule (52) dans lesquelles R' représente un groupement (b) dans lequel R₆ représente l'hydrogène,
* on traite l'acide ainsi formé :
   **soit :**
      a) avec un agent halogénant tel que le chlorure de thionyle pour obtenir un chlorure d'acyle que l'on estérifie avec un alcool de formule (18),
         **ou**
      b) avec un alcool de formule (18) en présence d'un acide fort comme catalyseur par exemple l'acide sulfurique pour obtenir les composés de formule (11) ou de formule (51) dans lesquelles R' représente un groupement (a),
   **soit :**
      avec un agent halogénant tel que le chlorure de thionyle pour obtenir un chlorure d'acyle que l'on amidifie avec un composé de formule (47) pour obtenir les composés de formule (11) ou de formule (52) dans lesquelles R' représente un groupement (c).

Les autres composés de départ ou intermédiaires intervenant dans les différents procédés décrits précédemment sont pour la plupart des composés connus ou pouvant être préparés par des méthodes connues.

On connaît déjà des dérivés de benzothiophène comportant une chaîne 1-alkénylbenzoyle et diversement substitués sur l'homocycle. De tels composés ont été décrits par exemple dans les brevets ou demandes de brevet US 5827876 ou W09701549 où ils sont présentés comme possédant des propriétés inhibitrices de la perte osseuse ou pour le traitement des symptômes de la ménopause ou encore pour le traitement de la restenose.

Or, on a maintenant découvert, dans le cadre de l'invention, que des dérivés de benzofurane ou benzothiophène comportant une chaîne aminoalkénylbenzoyle ainsi que d'autres groupements fixés sur l'hétérocycle par l'intermédiaire d'un atome de carbone, présentent de très intéressantes propriétés pharmacologiques notamment des propriétés antiarythmiques tout en offrant une très bonne stabilité métabolique, une solubilité très acceptable et une très bonne biodisponibilité par voie orale.

Les résultats de tests pharmacologiques effectués en vue de déterminer des propriétés des composés de l'invention sur le système cardiovasculaire sont répertoriés ci-dessous.

### I. Arythmies ventriculaires

Le but de ce test est de déterminer la capacité des composés de l'invention à assurer une protection contre les arythmies provoquées par reperfusion. A cet effet, on a utilisé la méthode rapportée par MANNING A.S. et coll. dans Circ. Res. 1984, 55 : 545-548 modifiée comme suit :
On anesthésie d'abord des rats, répartis en lots, avec du pentobarbital sodique (60mg/kg par voie intrapéritonéale) puis on les intube et les maintient sous respiration assistée.
On leur place ensuite une canule pour administration intraveineuse dans la veine jugulaire droite, on administre une dose intraveineuse du composé à étudier et 5 minutes plus tard, on place une boucle de ligature autour de l'artère coronaire descendante antérieure gauche et à proximité immédiate de son origine. On provoque alors l'occlusion de cette artère pendant 5 minutes par traction sur les extrémités de la ligature de manière à induire une reperfusion par relâchement de la tension.
On évalue alors les arythmies induites par cette reperfusion.
Un test analogue a été pratiqué par voie orale. Dans ce cas, le composé à étudier est administré 120 minutes avant la ligature de l'artère coronaire descendante antérieure gauche.
Les résultats de ces tests ont montré que les composés de l'invention protègent les animaux traités de manière significative allant jusqu'à 100% à des doses comprises entre 0,3 et 10mg/kg par voie intraveineuse et 10 à 90mg/kg par voie orale.

### II. Propriétés antiadrénergiques

Le but de ce test est de déterminer la capacité des composés de l'invention à réduire l'augmentation de la pression sanguine induite par la phényléphrine (effet anti-α) et l'accélération de la fréquence cardiaque induite par l'isoprénaline (effet anti-β) chez le chien préalablement anesthésié au pentobarbital et chloralose. On détermine d'abord pour chaque chien la dose de phényléphrine (5 ou 10µg/kg) qui provoque une augmentation de la pression artérielle comprise entre 25 et 40mm Hg et la dose d'isoprénaline (0,9 ou 1µg/kg) qui devra entraîner une augmentation de la fréquence cardiaque comprise entre 60 et 120 battements/minute.

On injecte alternativement toutes les 10 minutes les doses de phényléphrine et d'isoprénaline ainsi déterminées et après obtention de 2 réponses de référence successives, on administre une dose du composé à étudier par voie intraveineuse

### - Effet anti-α

On enregistre le pourcentage de réduction, par le composé de l'invention, de l'hypertension provoquée comparativement à l'hypertension de référence obtenue avant injection de ce composé (environ 100mm Hg).

### - Effet anti-β

On enregistre le pourcentage de réduction, par le composé à étudier, de

l'accélération provoquée de la fréquence cardiaque.
Les résultats de ces tests montrent qu'à des doses variant de 1 à 10 mg/kg, les composés de l'invention présentent des effets anti-α et/ou anti-β se traduisant par des réductions de l'hypertension provoquée et/ou de l'augmentation provoquée de la fréquence cardiaque, allant de 50% à presque 100%.

### III. Fibrillation auriculaire

Le but de ce test est d'évaluer l'efficacité des composés de l'invention vis-à-vis de la fibrillation auriculaire induite par stimulation permanente du nerf vague chez le chien anesthésié selon la méthode décrite dans Circulation 1993 ;88 : 1030-1044.

Les composés à étudier sont administrés aux doses cumulées de 3 et 10 mg/kg en perfusions intraveineuses lentes de 10 minutes pendant un épisode de fibrillation auriculaire soutenu.
A la dose de 10 mg/kg les composés de l'invention convertissent généralement 100% des fibrillations auriculaires en rythme sinusal et en préviennent la ré-induction dans 50 à 100% des cas. A cette dose, on observe des augmentations significatives de la période cardiaque ainsi que des périodes réfractaires effectives auriculaires pour différentes valeurs basales de la période cardiaque.

### IV. Effets inhibiteurs du système neuro-hormonal

Le but de ce test est de rechercher les effets inhibiteurs des composés de l'invention vis-à-vis des effets vasoconstricteurs induits par différents peptides tels que la noradrénaline (NA), l'angiotensine II (A-II), l'arginine vasopressine (AVP), le neuropeptide Y (NPY) et l'endothéline (ET) et également vis-à-vis des effets tachycardes induits par l'isoprénaline (Iso) chez le rat vigile.

Chez des rats mâles Sprague Dawley d'environ 300g, on implante, 24 heures avant le test, un cathéter artériel (artère carotide droite) pour la mesure de la pression artérielle et un cathéter veineux (veine jugulaire droite) pour l'injection des produits à étudier. Le lendemain, on place les rats dans des boîtes cylindriques et on relie le cathéter artériel à un capteur de pression par l'intermédiaire d'un joint tournant sur balancier. Ce capteur de pression est lui-même relié à un polygraphe pour enregistrement de la pression artérielle.

On recherche alors l'action des composés de l'invention, par voie intraveineuse, vis-à-vis des effets vasoconstricteurs induits par la NA (1 µg/kg), l'A-II (100 µg/kg) et l'AVP (40 µg/kg) aux doses respectives de soit 3, 10 et 30 mg/kg soit 1,3 à 10 mg/kg et uniquement à la dose de 10 mg/kg vis-à-vis des effets vasoconstricteurs induits par le NPY (6 µg/kg) et l'ET (0,5 µg/kg) ou des effets tachycardies induits par l'Iso (1 µg/kg).

On solubilise d'abord les différents agonistes peptiques dans du sérum physiologique à 0,9% et le composé à étudier dans un solvant approprié. On injecte ensuite ces peptides en bolus sous un volume de 0,05 ml/kg et ce, 30 et 10 minutes avant l'administration intraveineuse de 0,1 ml/kg d'une solution du composé à étudier ou de solvant. On répète ensuite ces injections de peptide 10, 30, 60 et 120 minutes après l'administration du composé à étudier. En fonction de la durée d'action du composé à tester, on peut éventuellement prolonger ces injections toutes les 30 minutes sans jamais dépasser 5 heures au total.

On évalue alors les variations de la pression artérielle après administration d'un peptide donné en mesurant, à différents temps, la différence entre l'effet maximal induit par l'agoniste peptide et la valeur basale de la pression artérielle.

Les résultats obtenus montrent que la NA, l'A-II, l'AVP, le NPY et l'ET induisent des augmentations respectives de la pression artérielle de 45 ±3, 40±3, 30±2 et 34±4 mmHg et l'Iso une augmentation de la fréquence cardiaque de 209±7 battements par minute.

En outre, on observe que les composés de l'invention antagonisent d'une manière dose-dépendante les effets vasoconstricteurs induits par la NA, l'A-II et l'AVP. Ils antagonisent également les effets induits par le NPY et par l'ET et l'augmentation de la fréquence cardiaque induite par l'Iso. Aux doses les plus élevées, l'inhibition maximale obtenue après 15 minutes varie entre 40 et 80% et la durée d'action est au moins supérieure ou égale à 30 minutes.

### V. Toxicité

La toxicité des composés de l'invention s'est révélée compatible avec leur utilisation en thérapeutique.

Les compositions pharmaceutiques selon l'invention peuvent être présentées sous toute forme convenant à l'administration en thérapie humaine ou vétérinaire. Par exemple les compositions pharmaceutiques de la présente invention peuvent être formulées pour l'administration orale, sublinguale, sous-cutanée, intramusculaire, intraveineuse, transdermique ou rectale. Pour ce qui concerne l'unité d'administration, celle-ci peut prendre la forme, par exemple, d'un comprimé, d'une dragée, d'une capsule, d'une gélule, d'une poudre, d'une suspension, d'un sirop ou encore de granules pour l'administration orale, d'un suppositoire pour l'administration rectale ou d'une solution ou suspension pour l'administration parentérale.

Les compositions pharmaceutiques de l'invention pourront comprendre, par unité d'administration, par exemple de 50 à 500mg en poids d'ingrédient actif pour l'administration orale, de 50 à 200mg d'ingrédient actif pour l'administration rectale et de 50 à 150mg d'ingrédient actif pour l'administration parentérale.

Suivant la voie d'administration choisie, les compositions pharmaceutiques ou vétérinaires de l'invention seront préparées en associant au moins un des composés de formule (1) ou un sel pharmaceutiquement acceptable de ce composé avec un excipient approprié, ce dernier pouvant être constitué par exemple d'au moins un ingrédient sélectionné parmi les substances suivantes : lactose, amidons, talc, stéarate de magnésium, polyvinylpyrrolidone, acide alginique, silice colloïdale, eau distillée, alcool benzylique ou agents édulcorants.

Lorsqu'il s'agit de comprimés, ceux-ci peuvent être traités de telle sorte qu'ils présentent une activité prolongée ou retardée et qu'ils libèrent d'une façon continue une quantité prédéterminée de principe actif.

Les Exemples, non limitatifs suivants, illustrent la préparation des composés et compositions de l'invention :

### EXEMPLE 1

### Chlorhydrate de 2-butyl-3-[4-[(E)-3-(dibutylamino)-1-propenyl]benzoyl]-1-benzofurane-5-carboxylate d'isopropyle

### A. 2-Butyl-3-(4-trifluorométhanesulfonyloxy-benzoyl)-1-benzofurane-5-carboxylate d'isopropyle

On introduit 11,7g (30,8mmoles) de 2-butyl-3-(4-hydroxy-benzoyl)-1-benzofurane-5-carboxylate d'isopropyle dans 100ml de dichlorométhane contenant 2,67g (33,8mmoles) de pyridine.

On ajoute alors, entre 0° et 5°C, 9,54g (33,8mmoles) d'anhydride trifluorométhanesulfonique dans 50ml de dichlorométhane. On agite le milieu réactionnel à température ambiante durant 1 heure 30 minutes puis on concentre à sec. On reprend avec de l'éther diéthylique puis lave avec de l'eau, de l'acide chlorhydrique dilué, de l'eau, une solution d'hydrogénocarbonate sodique, de l'eau puis une solution de chlorure de sodium. On concentre à sec puis on triture dans l'heptane. On essore alors sur verre fritté.

De cette manière, on obtient 11 g de composé désiré.
Rendement : 69,6%
P.F. : 100-101°C

### B. Chlorhydrate de 2-butyl-3-[4-[(E)-3-(dibutylamino)-1-propenyl]benzoyl]-1-benzofuranne-5-carboxylate d'isopropyle

Sous argon, on dissout, dans 100 ml de dioxane, 4,17g (8,1mmoles) de composé obtenu au paragraphe précédent, 3,73g (1 équivalent) de (E)-1-tributylétain-3-(dibutylamino)-1-propène, 0,96g de chlorure de lithium et 1,08g de tétrakis (triphénylphosphine)palladium. On porte au reflux durant 3 heures, on évapore et on reprend dans l'eau. On extrait avec de l'éther diéthylique, et on lave avec de l'eau et une solution saturée de chlorure de sodium. On purifie alors par chromatographie sur silice (éluant : dichlorométhane/méthanol/ammoniaque 100/3/0,2), ce qui fournit 4,95g (rendement : 100%) de composé désiré sous forme basique.

On dissout alors, dans l'éther diéthylique, 4,90g du composé basique ainsi obtenu et on ajoute une solution de chlorure d'hydrogène dans l'éther diéthylique. On laisse ensuite cristalliser dans l'éther diéthylique.

De cette manière, on obtient 3 g de composé désiré.
Rendement : 64%
P.F. : 147-149°C

En utilisant le même procédé que précédemment, on a préparé les composés suivants :
Chlorhydrate de 2-butyl-6-méthyl-3-[4-[(E)-3-(dibutylamino)propyl]benzoyl]-1-benzofurane-5-carboxylate de méthyle (**Exemple 2**)
Rendement : 58,4%
Spectre R.M.N. (résonance magnétique nucléaire) (200 MHz)
Solvant : DMSO (diméthylsulfoxyde) à 2,5 ppm
   DOH à 3,33 ppm

- δ (ppm) :: 0,6 à 1 ; massif ; 9H, 3CH₃
1 à 1,9 ; massif ; 12H, 6CH₂
2,6 ; singulet; 3H, CH₃
2,7 ; triplet ; 2H, CH₂
3 ; multiplet; 4H, 2CH₂N
3,75 ; singulet; 3H, OCH₃
3,9 ; multiplet; 2H, NCH₂
6,6 ; doublet de triplet; 1H, CH
7 ; doublet; 1H, CH
7,4 à 8,1 ; massif ; 6H, ¹H aromatique
10,8 ; singulet élargi ; 1H, NH⁺

### EXEMPLE 3

### Oxalate de 2-butyl-3-[4-[(Z)-3-(dibutylamino)-1-propenyl]benzoyl]-1-benzofuranne-5-carboxylate d'isopropyle

### A. 2-Butyl-3-[4-[(Z)-3-(dibutylamino)-1-propynyl]benzoyl]-1-benzofurane-5-carboxylate d'isopropyle

Sous argon, on introduit 5g (9,75mmoles) de 2-butyl-3-(4-trifluorométhanesulfonyloxy-benzoyl)-1-benzofurane-5-carboxylate d'isopropyle dans 50ml de N,N-diméthylformamide puis on ajoute 1, 63g de 3-(dibutylamino)-1-propyne, 6,72ml de triéthylamine, 0,341g de dichloro bis-(triphénylphosphine)palladium et 0,094g d'iodure cuivreux. On chauffe aux environs de 90°C pendant 3 heures puis on évapore et on reprend le résidu avec de l'éther diéthylique. On lave avec de l'eau et une solution de chlorure de sodium puis on purifie par chromatographie sur silice (éluant : dichlorométhane/méthanol 99/1).

De cette manière, on obtient 4,84g de composé désiré.
Rendement : 91%

### B. Oxalate de 2-butyl-3-[4-[(Z)-3-(dibutylamino)-1-propenyl]benzoyl]-1-benzofurane-5-carboxylate d'isopropyle

On introduit 4,8g (8,8mmoles) de composé obtenu précédemment dans 100ml de toluène. On ajoute une spatule de charbon animal, on agite et on filtre sur terre de diatomées. On rince avec 150ml de toluène, on ajoute à ce filtrat, 0,480g de charbon palladié de Lindlar puis on hydrogène à 25°C et à pression normale.
On suit l'évolution de la réaction par chromatographie sur couche mince et on ajoute des portions de 0,480g de charbon palladié jusqu'à la fin de cette réaction. On filtre sur terre de diatomées, rince et purifie par chromatographie sur silice (éluant : dichlorométliane/méthanol/ammoniaque 20% : 98/2/0,2), ce qui fournit 2,05g (rendement : 48,3%) de composé désiré sous forme de base libre. On dissout alors, dans 20ml de méthanol, 1,94g de composé basique ainsi obtenu, puis on ajoute une solution de 0,328g d'acide oxalique dans 20 ml de méthanol. On évapore et reprend le résidu dans l'éther diéthylique. On filtre, lave à l'éther diéthylique et sèche sous vide.

De cette manière, on obtient 1,649g de composé désiré.
Rendement : 73%
P.F. :78-80°C

En utilisant le même procédé que celui décrit précédemment, on a préparé le composé suivant :
Chlorhydrate de 2-butyl-6-méthyl-3-[4-[(Z)-3-(dibutylamino)-1-propenyl]benzoyl]-1-benzofurane-5-carboxylate d'isopropyle. (**Exemple 4**) P.F.: 129°C

### EXEMPLE 5

### Oxalate de l'acide 2-butyl-3-[4-[(Z)-3-(dibutylamino)-1-propenyl]benzoyl]-1-benzofurane-5-carboxylique

Dans 25ml de dioxane, on introduit 2,26g de 2-butyl-3-[4-[(Z)-3-(dibutylamino)-1-propenyl]benzoyl]-1-benzofurane-5-carboxylate de méthyle et 0,360g (2 équivalents) d'hydroxyde de sodium puis on ajoute 5ml d'eau et 5ml de méthanol. On agite durant 24 heures à température ambiante puis on évapore. On reprend dans l'eau et on acidifie jusqu'à pH = 5 à 6 avec de l'acide chlorhydrique dilué.

On extrait avec de l'acétate d'éthyle et on lave avec une solution saturée de chlorure de sodium. On purifie alors par chromatographie sur silice (éluant : dichlorométhane/ méthanol 100/7), ce qui fournit 1,45g (rendement : 66%) du composé désiré sous forme de base libre.

On introduit alors dans l'acétone 2,25g du composé basique ainsi obtenu et 0,414g d'acide oxalique. On évapore et on laisse cristalliser dans l'éther diéthylique.

De cette manière, on recueille 2,12g du composé désiré.
Rendement : 79,5%
P.F. : 89-92C°

### EXEMPLE 6

### Oxalate de 2-butyl-3-[4-[(Z)-3-(dibutylamino)-1-propenyl]benzoyl]-1-benzofurane-5-carboxamide

On introduit 6,79g (13,9mmoles) d'acide 2-butyl-3-[4-[(Z)-3-(dibutylamino)-1-propenyl]benzoyl]-1-benzofurane-5-carboxylique dans 100ml de dichlorométhane puis on ajoute 3,13g (15,2mmoles) de dicyclohexylcarbodiimide et 2,05g (15,2mmoles) d' hydroxybenzotriazole. On agite durant 0,5 heure à température ambiante, on refroidit alors à 5°C et on ajoute 2,6 ml d'ammoniaque à 20%. On agite à nouveau à température ambiante durant 18 heures puis on refroidit. On essore sur verre fritté, on lave le filtrat avec de l'eau, une solution d'hydrogénocarbonate de sodium puis avec de l'eau. On sèche, concentre et purifie par chromatographie sur silice (éluant : dichlorométhane/méthanol/ammoniaque 100/3/0,1), ce qui fournit 2,92g (rendement: 43%) de composé désiré sous forme basique.

On introduit ensuite 2,90g (5,94mmoles) de composé basique ainsi obtenu, dans une quantité nécessaire de méthanol pour obtenir une dissolution totale puis on ajoute 0,535g (5,9mmoles) d'acide oxalique. On concentre à sec, triture dans l'éther diéthylique, essore puis sèche.

De cette manière, on recueille 2,36g de composé désiré sous forme d'un solide amorphe.
Spectre R.M.N. (200 MHz)
Solvant: DMSO à 2,5 ppm
- δ (ppm) :: 0,6 à 0,9 ; massif ; 9H, 3CH₃
1 à 1,8 ; massif ; 12H, 6CH₂
2,75 ; triplet; 2H, CH₂
2,9 ; multiplet; 4H, 2NCH₂
3,95 ; doublet ; 2H, NCH₂
6 ; doublet de triplet; 1 H ; CH
6,9 ; doublet; 1 H, CH
7,05 à 8,1 ; massif ; 9H, 7 ¹H aromatique, NH₂

### EXEMPLE 7

### Oxalate de 2-butyl-3-[4-[(Z)-3-(dibutylamino)-1-propenyl]benzoyl]-N,N-diméthyl-1-benzofurane-5-carboxamide

### A. 2-Butyl-3-(4-hydroxy-benzoyl)-N,N- diméthyl-1-benzofurane-5-carboxamide

Dans 300ml de 1,2-dichloroéthane, on introduit 6g d'acide 2-butyl-3-(4-hydroxy-benzoyl)-1-benzofurane-5-carboxylique et 10ml de chlorure de thionyle. On porte à reflux durant 7 heures puis on concentre à sec. On dissout alors ce chlorure dans 100ml de dichlorométhane et, à 5°C on sature par la quantité nécessaire de diméthylamine sous forme de gaz. On agite alors à température ambiante pendant 72 heures. On lave avec de l'eau, de l'acide chlorhydrique dilué, de l'eau puis une solution saturée de chlorure de sodium. On purifie ensuite par chromatographie sur silice (éluant : dichlorométhane/méthanol 97/3).

De cette manière, on obtient 5,2g du composé désiré.
Rendement : 80,9% (par rapport au composé 5-carboxylique de départ)

### B. 2-Butyl-3-(4-trifluorométhanesulfonyloxy-benzoyl)-N,N-diméthyl-1-benzofurane-5-carboxamide

On introduit dans 50ml de dichlorométhane, 5,1g (14 mmoles) de composé obtenu à l'étape précédente et on ajoute 2,52ml de pyridine. On additionne alors, à une température inférieure à 10°C, 5,26ml (31,3mmoles) d'anhydride trifluorométhanesulfonique dans 30ml de dichlorométhane. On agite à température ambiante pendant 4 heures, on concentre à sec et on reprend avec de l'acétate d'éthyle. On lave avec de l'eau, de l'acide chlorhydrique dilué, de l'eau, une solution d'hydrogénocarbonate de sodium, de l'eau et une solution de chlorure de sodium. On purifie alors par chromatographie sur couche mince (éluant : dichlorométhane/acétate d'éthyle 95/ puis 90/10 puis 80/20).

De cette manière, on obtient 5,358g de composé désiré.
Rendement : 76,9%

### C. 2-Butyl-3-[4-[3-(dibutylamino)-1-propynyl]benzoyl]-N,N-diméthyl-1-benzofurane-5-carboxamide

Sous argon, on introduit dans 60ml de N,N-diméthylformamide, 5,338g (10,73mmoles) de composé obtenu à l'étape précédente puis on ajoute 1,8g (10,73mmoles) de 3-(dibutylamino)-1-propyne. On additionne ensuite 7,39ml de triéthylamine, 0,376g de dichloro bis-(triphénylphosphine) palladium puis 0,104g d'iodure cuivreux. On chauffe à 90°C durant 4 heures, on refroidit et on dilue avec de l'eau. On extrait à l'éther diéthylique, lave la phase organique avec de l'eau puis avec une solution de chlorure de sodium. On purifie ensuite par chromatographie sur silice (éluant: dichlorométhanelméthanol 98/2).

De cette manière, on obtient 3,339g de composé désiré.
Rendement : 60,4%

### D. Oxalate de 2-butyl-3-[4-[(Z)-3-(dibutylamino)-1-propenyl]benzoyl] -N,N-diméthyl-1-benzofurane-5-carboxamide

On dissout dans 100ml de toluène 3,32 g de composé obtenu à l'étape précédente puis on ajoute 1,32 g de charbon palladié de Lindlar et on hydrogène à température ambiante et à pression normale. On filtre sur terre de diatomées et on évapore le solvant sous pression réduite. On purifie ensuite le résidu par chromatographie sur silice (éluant : dichlorométhane/méthanol/ammoniaque 98/2/0,1), ce qui fournit 1,105 g (rendement : 33,1 %) de composé désiré sous forme basique.
On introduit alors 1,095g (2,12mmoles) de composé basique ainsi obtenu, dans la quantité nécessaire de méthanol pour obtenir une dissolution totale puis on ajoute 0,191g (2,12mmoles) d'acide oxalique. On concentre à sec et triture dans l'éther diéthylique. On essore puis on sèche.

De cette manière, on obtient 1,053g de composé désiré.
Rendement : 81,8%
Spectre R.M.N. (200 MHz)
Solvant: DMSO à 2,5 ppm
- δ (ppm) :: 0,7 à 1,1 ; massif ; 9H, 3CH₃
1,1 à 1,9 ; massif; 12H, 6CH₂
2,7 à 3,2 ; massif ; 12H, 3CH₂, 2NCH₃
4,15 ; doublet ; 2H, NCH₂
6,1 ; doublet de triplet ; 1 H, CH
7 ; doublet ; 1 H, CH
7,3 à 8 ; massif ; 7H ; ¹H aromatique

### EXEMPLE 8

### Oxalate de 2-butyl-3-[3-[(Z)-3-(dibutylamino)-1-propenyl]benzoyl]-1-benzofurane-5-carboxylate d'isopropyle

### A. 2-Bulyl-3-(3-méthoxy-benzoyl)-1-benzofurane-5-carboxylate de méthyle

Dans 300ml de 1,2-dichloroéthane, on introduit 14,8g (63,7mmoles) de 2-butyl-1-benzofurane-5-carboxylate de méthyle et 21,6g (127mmoles) de chlorure de 3-méthoxybenzoyle puis on ajoute par fractions 21,6g (127,4mmoles) de chlorure ferrique et on agite à température ambiante durant environ 8 heures. On verse sur un mélange eau/glace, on décante et on extrait avec du dichlorométhane. On lave avec de l'eau, une solution diluée d'hydrogénocarbonate de sodium puis avec de l'eau.

De cette manière, on obtient 20,52g de composé désiré.
Rendement : 87,9%

### B. 2-Butyl-3-(3-hydroxy-benzoyl)-1-benzofurane-5-carboxylate de méthyle

Dans 500ml de toluène, on introduit 21,73g (59,3mmoles) de composé obtenu à l'étape précédente et on ajoute 23,72g (178mmoles) de chlorure d'aluminium. On chauffe à 60°C pendant 4 heures, on refroidit et on décante. On dissout dans le tétrahydrofurane l'insoluble présent dans le toluène, on ajoute un mélange d'eau et de glace et on extrait avec de l'acétate d'éthyle. On réunit les extraits organiques, on lave plusieurs fois avec de l'eau et avec une solution de chlorure de sodium. On sèche et on concentre.

De cette manière, on obtient 22g de composé désiré sous forme brute.

### C. Acide 2-butyl-3-(3-hydroxy-benzoyl)-1-benzofurane-5-carboxylique

Dans 300ml de dioxane, on introduit 19g (53,9mmoles) de composé obtenu à l'étape précédente, puis on ajoute 4,31g (107,8mmoles) d'hydroxyde de sodium dans 150ml d'eau. On agite à température ambiante pendant 5 heures et on concentre à sec. On reprend par de l'eau et on acidifie par de l'acide chlorhydrique dilué. On extrait alors avec de l'acétate d'éthyle.

De cette manière, on obtient 23g de composé désiré sous forme brute.

### D. 2-Butyl-3-(3-hydroxy-benzoyl)-1-benzofurane-5-carboxylate d'isopropyle

Dans 500ml d'isopropanol, on introduit 23,5g de composé brut obtenu à l'étape précédente et 5ml d'acide sulfurique. On porte à reflux pendant environ 12 heures et on concentre à sec. On reprend avec de l'acétate d'éthyle et lave successivement avec de l'eau, une solution d'hydrogénocarbonate de sodium, de l'eau et une solution de chlorure de sodium. On purifie ensuite par chromatographie sur silice (éluant : dichlorométhane/ méthanol 99/1 puis 98/2).

De cette manière, on obtient 13,3g de composé désiré.

### E. 2-Butyl-3-(3-trifluorométhanesulfonyloxy-benzoyl)-1-benzofurane-5-carboxylate d'isopropyle

Dans 100ml de dichlorométhane, on introduit 10g (26,3mmoles) de composé obtenu à l'étape précédente puis on ajoute 2,33ml (28,9mmoles) de pyridine. On ajoute alors, entre 5° et 10°C, 4,87ml (28,9mmoles) d'anhydride trifluorométhanesulfonique dans 50ml de dichlorométhane.

On agite à température ambiante pendant environ 8 heures et on concentre à sec. On reprend avec de l'acétate d'éthyle et on lave avec de l'eau, de l'acide chlorhydrique dilué, une solution d'hydrogénocarbonate de sodium, de l'eau et finalement avec une solution de chlorure de sodium.

On purifie ensuite par chromatographie sur silice (éluant : dichlorométhane).

De cette manière, on obtient 8,31 g de composé désiré. Rendement : 61,6%

### F. 2-Butyl-3-[3-[3-(dibutylamino)-1-propynyl]benzoyl]-1-benzofurane-5-carboxylate d'isopropyle

Sous argon, on introduit, dans 80ml de N,N-diméthylformamide, 8,3g (16,2mmoles) de composé obtenu à l'étape précédente puis on ajoute 2,71g (16,2mmoles) de 3-dibutylamino-1-propyne, 11,16ml de triéthylamine, 0,566g de dichloro bis-(triphénylphosphine) palladium et 0,156g d'iodure cuivreux. On chauffe vers 90°C pendant 6 heures. On verse le milieu réactionnel dans de l'eau puis on extrait avec de l'éther diéthylique. On lave avec de l'eau et on chromatographie sur silice (éluant : dichlorométhane/méthanol 99/1)

De cette manière, on obtient 5,2g de composé désiré. Rendement ; 60,6%

### G. Oxalate de 2-butyl-3-[3-[(Z)-3-(dibutylamino)-1-propenyl]benzoyl]-1-benzofurane-5-carboxylate d'isopropyle

On introduit, dans du toluène, 5,2g (10,1mmoles) de composé obtenu à l'étape précédente et on hydrogène la solution obtenue en présence de 0,520g de charbon palladié de Lindlar. On suit la réaction par chromatographie sur couche mince et on additionne des fractions de 0,520g de charbon palladié selon le degré d'avancement de la réaction. On essore sur terre de diatomées et on concentre. On purifie alors par chromatographie sur silice (éluant : dichlorométhanelméthanol 97/3), ce qui fournit 2,586g de composé désiré sous forme basique.

On introduit alors 2,56g (48,1mmoles) de composé basique obtenu à l'étape précédente, dans la quantité nécessaire de méthanol pour obtenir la dissolution totale. On ajoute ensuite 0,433g (48,1mmoles) d'acide oxalique et on concentre à sec. On triture dans l'éther diéthylique, on essore et on sèche.

De cette manière, on recueille 2,516g de composé désiré.
P. F. : 76-78°C

### EXEMPLE 9

### Oxalate de 2-butyl-3-[4-[(Z)-3-(dibutylamino-1-propenyl]benzoyl-5-(1H-tétrazol-5-yl)-1-benzofurane

On introduit 3,32g (7,06mmoles) de 2-butyl-3-[4-[(Z)-3-(dibutylamino)-1-propenyl]benzoyl]-1-benzofurane-5-carbonitrile dans 70 ml de toluène puis on ajoute 3g (8,9mmoles) d'azide de tributylétain. On porte à reflux pendant 3 jours et demi puis on concentre à sec. On purifie ensuite par chromatographie sur silice (éluant : dichlorométhane/méthanol 90/10), ce qui fournit 3,105g de composé désiré sous forme basique.

On introduit ensuite 3,034g (5,91 mmoles) de composé basique ainsi obtenu, dans la quantité nécessaire de méthanol pour obtenir la dissolution puis on ajoute 0,532g (5,91mmoles) d'acide oxalique. On concentre, triture dans l'éther diéthylique et essore.

De cette manière, on obtient 2,99g de composé désiré.
P.F. :173-174°C

### EXEMPLE 10

### Chlorohydrate de 2-butyl-3-[4-[(Z)-3-butylamino-1-propenyl]benzoyl]-1-benzofurane-5-carboxylate d'isopropyle

### A. 3-(4-[3-[(tert-butoxycarbonyl)(butyl)amino]-1-propynyl]benzoyl)-2-butyl-1-benzofurane-5-carboxylate d'isopropyle

Dans 180ml de N,N-diméthylformamide, contenant 18ml de triéthylamine, on mélange 11,04g (22mmoles) de 2-butyl-3-(4-trifluorométhanesulfonyloxy-benzoyl)-1-benzofurane-5-carboxylate d'isopropyle, 4,55g (22mmoles) de 3-(N-butyl-N-tert-butoxycarbonyl)amino-1-propyne, 0,875g de dichloro bis-(triphénylphosphine)palladium et 0,312g d'iodure cuivreux. Sous argon, on chauffe à 90 °C pendant 3 heures puis on verse sur un mélange eau/glace. On extrait avec de l'éther diéthylique puis on lave avec de l'eau et une solution saturée de chlorure de sodium. On purifie ensuite par chromatographie sur silice (éluant : dichlorométhane/ acétate d'éthyle 100/2)

De cette manière, on obtient 9,73g de composé désiré.
Rendement : 77%

### B. Chlorhydrate de 2-butyl-3-[4-[3-butylamino-1-propynyl]benzoyl]-1-benzofurane-5-carboxylate d'isopropyle

Dans 20ml d'une solution d'acide chlorhydrique/acétate d'éthyle 3M, on introduit 0,940g de composé obtenu à l'étape précédente et on agite durant 2 heures à température ambiante. On évapore et on cristallise dans l'éther diéthylique.

De cette manière, on recueille 0,706g de composé désiré.
Rendement : 84,5%
P.F. : 166-167°C

### C. Chlorhydrate de 2-butyl-3-[4-[(Z)-3-butylamino-1-propenyl]benzoyl]-1-benzofurane-5-carboxylate d'isopropyle

On introduit, dans 20ml d'éthanol, 0,680g de composé obtenu à l'étape précédente et on hydrogène en présence de 0,090g de charbon palladié de Lindlar. On ajoute alors successivement 0,120 ; 0,160 et 0,200g de charbon palladié selon le degré d'avancement de la réaction. On filtre ensuite sur terres de diatomées et on purifie par chromatographie sur silice (éluant : dichlorométhane/ méthanol/ ammoniaque 100/3/0,2), ce qui fournit 0,340g (rendement : 67%) de composé désiré sous forme basique.

On introduit alors 0,320g de composé basique, ainsi obtenu, dans de l'éther diéthylique et on ajoute une solution de chlorure d'hydrogène dans l'éther diéthylique. On évapore et on cristallise dans l'éther diéthylique.

De cette manière, on recueille 0,310g de composé désiré.
Rendement : 89%
P.F. : 175-176°C

### EXEMPLE 33

### Oxalate de 2-butyl-3-(4-[(E)-3-(dibutylamino)-1-propenyl]benzoyl)-1-benzofuran-5-carboxylate de 2-(diméthylamino)-éthyle

### A. 2-Butyl-3-(4-hydroxy-benzoyl)-benzofuran-5]-carboxylate de 2-diméthylamino-éthyle

On mélange 11, 63 g (0,0344 moles) d'acide 2-butyl-3-(4-hydroxy-benzoyl)-benzofuran-5-carboxylique, 250 ml de N,N-diméthylformamide et 5,58 g (0,0344 moles) de carbonyldiimidazole. On chauffe à 40° C pendant 2 heures puis on ajoute 5,23 g (0,0344 moles) de DBU ( 1,8-diazabicyclo[5.4.0]undec-7-ène) et 6,13 g (0,0687 moles) de diméthylamino-2-éthanol. On agite en chauffant à 40° C pendant 18 heures. On concentre à sec puis on reprend à l'acétate d'éthyle et on lave à l'eau. On purifie par chromatographie sur silice (éluant : dichlorométhane/méthanol à 95/5).

De cette manière, on obtient 9,35 g du composé désiré. Rendement : 66,4 %

### B. 2-Butyl-3-(4-trifluoromethanesulfonyloxy-benzoyl)-benzofuran-5-carboxylate de 2-dimethylamino-ethyl

On mélange 10,49 g (0,0256 moles) du composé obtenu à l'étape précédente A, 150 ml d'EtCl₂ (1,2-dichloroéthane), (4,46) g soit (0,056) moles de pyridine. A ce mélange, on ajoute, à une température inférieure à 10° C, (15,88) g soit (0,056) moles d'anhydride triflique dissous dans 300 ml de dichlorométhane. On agite pendant 5 jours à température ambiante, on concentre à sec puis on reprend dans l'eau. On ajoute ensuite du NaHCO₃ jusqu'à pH basique. On extrait à l'acétate d'éthyle et on lave à l'eau. On purifie par chromatographie sur silice (éluant : dichlorométhane/méthanol à 90/10).

De cette manière, on obtient 3,52 g du composé désiré.
Rendement : 25,4 %

### C. 2-butyl-3-(4-[(E)-3-(dibutylamino)-1-propenyl]benzoyl)-1-benzofuran-5-carboxylate de 2-(diméthylamino)-éthyle

On mélange 4,97 g (0,00918 moles) du composé obtenu à l'étape précédente B, 150 ml de dioxanne, 4, 23 g (0,00918 moles) de (E)-1-tributylétain-3-(dibutylamino)-1-propène, 1,084 g de chlorure de lithium et 1,224 g de tétrakis(triphénylphosphine)-palladium. On porte au reflux pendant 5 heures. On concentre à sec et on reprend à l'acétate d'éthyle puis on lave à l'eau. On purifie par chromatographie sur silice (éluant : dichlorométhane/méthanol à 90/10 puis dichlorométhane/méthanol/NH₄OH à 95/5/0,2.

De cette manière, on obtient 0,84 g du composé désiré.
Rendement :16,3 %.

### D. Oxalate de 2-butyl-3-(4-[(E)-3-(dibutylamino)-1-propenyl]benzoyl)-1-benzofuran-5-carboxylate de 2-(diméthylamino)-éthyle

On mélange de 835 mg (0,00149 moles) du composé obtenu à l'étape C précédente, une quantité suffisante de méthanol pour une dissolution totale et 268 mg (0,00298 moles) d'acide oxalique. On concentre ensuite à sec et on reprend par de l'éther. On essore et on sèche.

De cette manière, on obtient 890 mg du composé désiré.
Rendement : 80,6 %.

En utilisant les procédés décrits aux exemples précédents, on a préparé les composé listes ci-après :

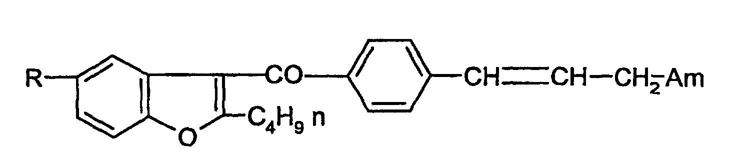

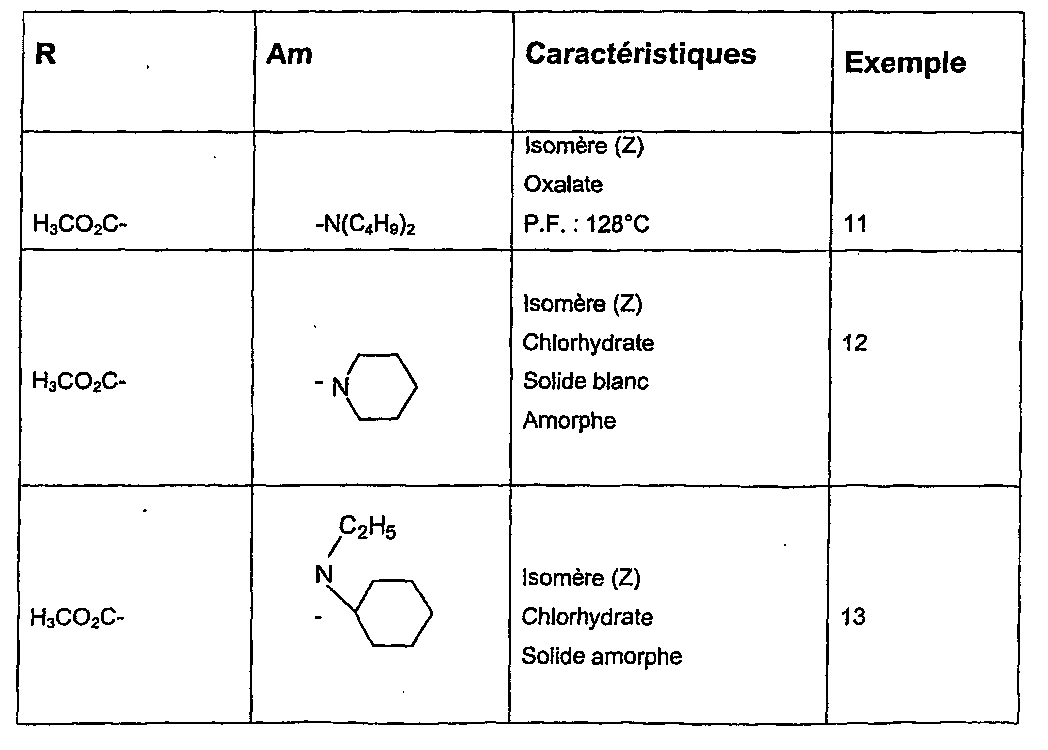

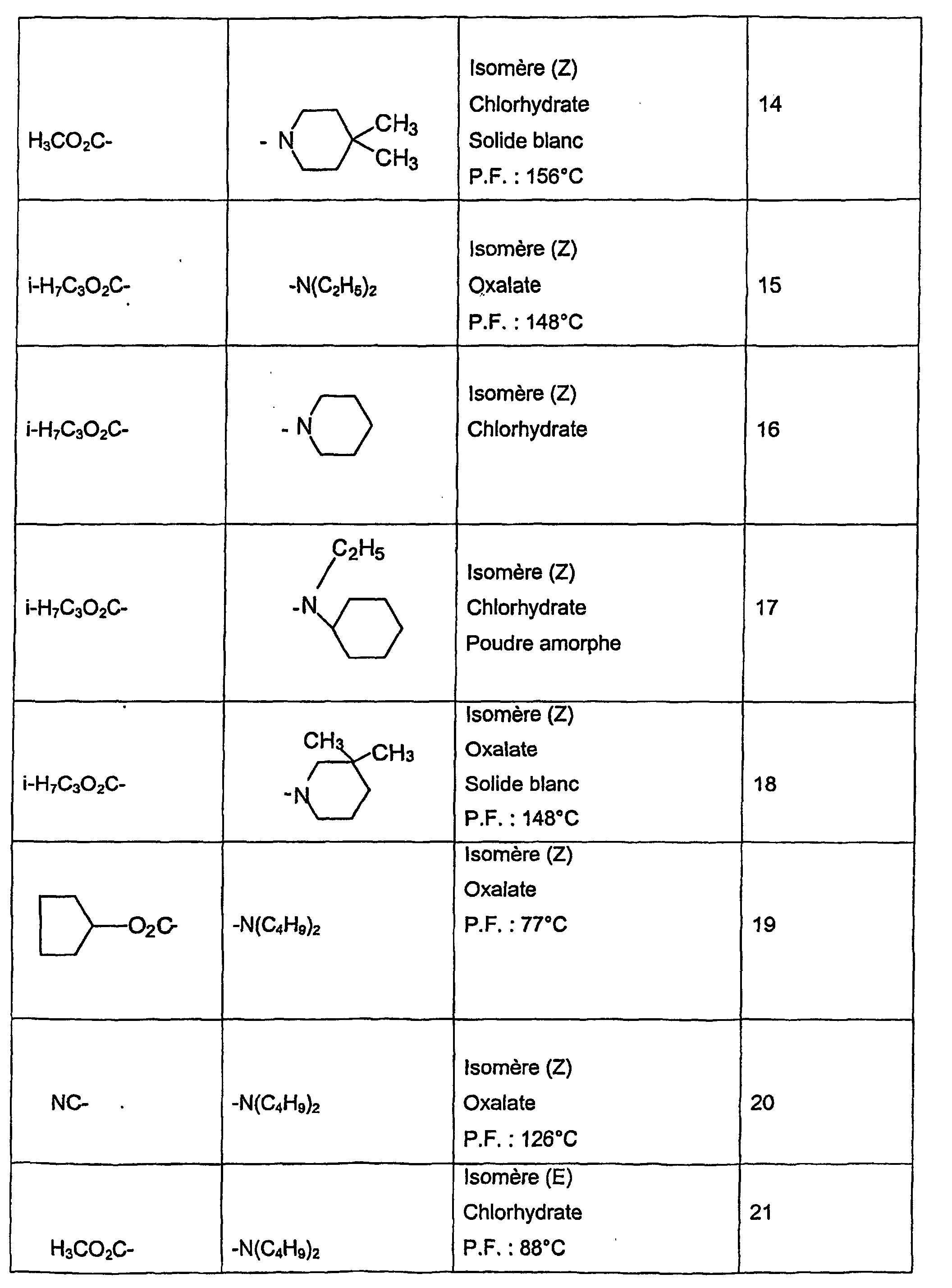

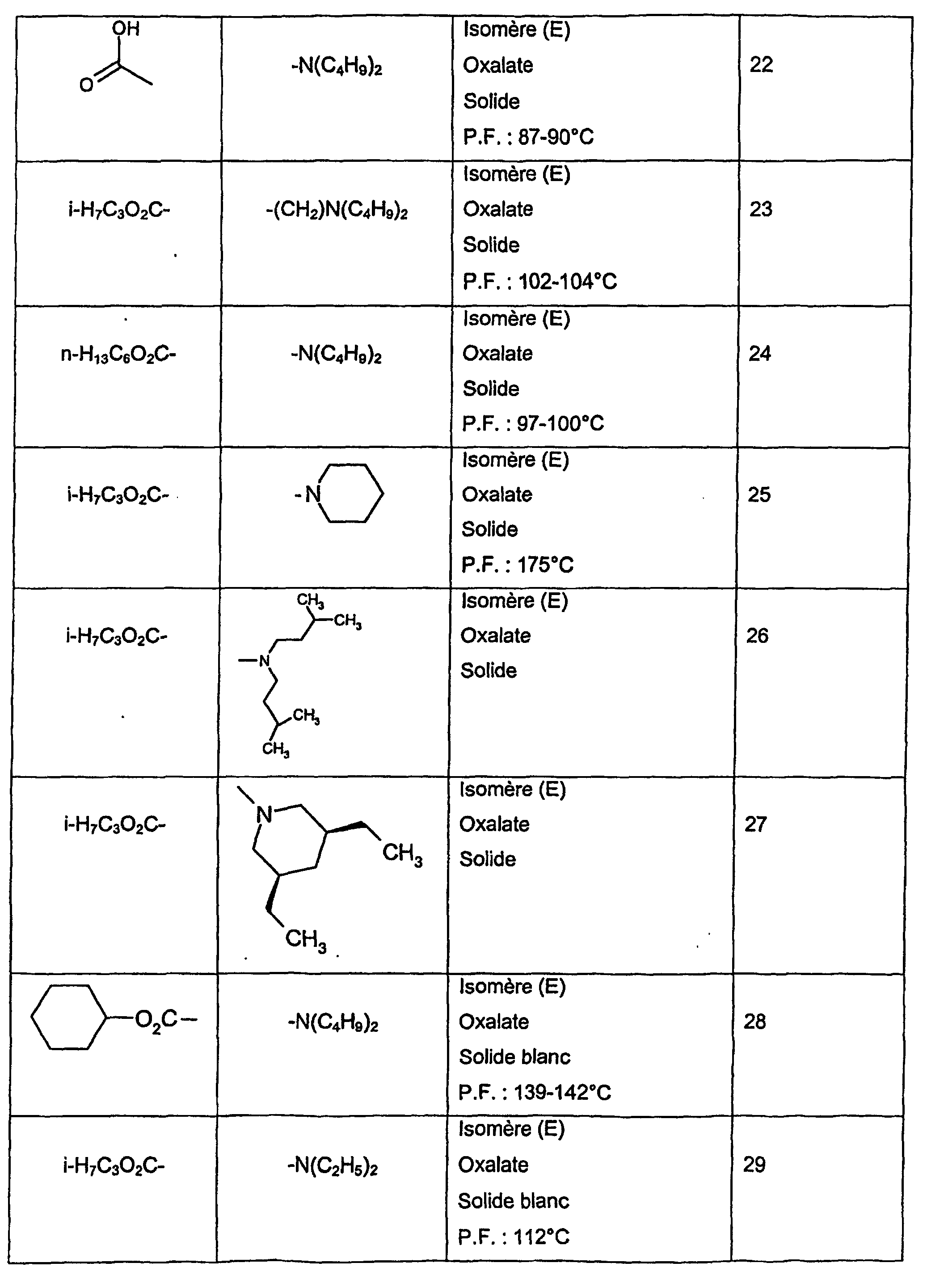

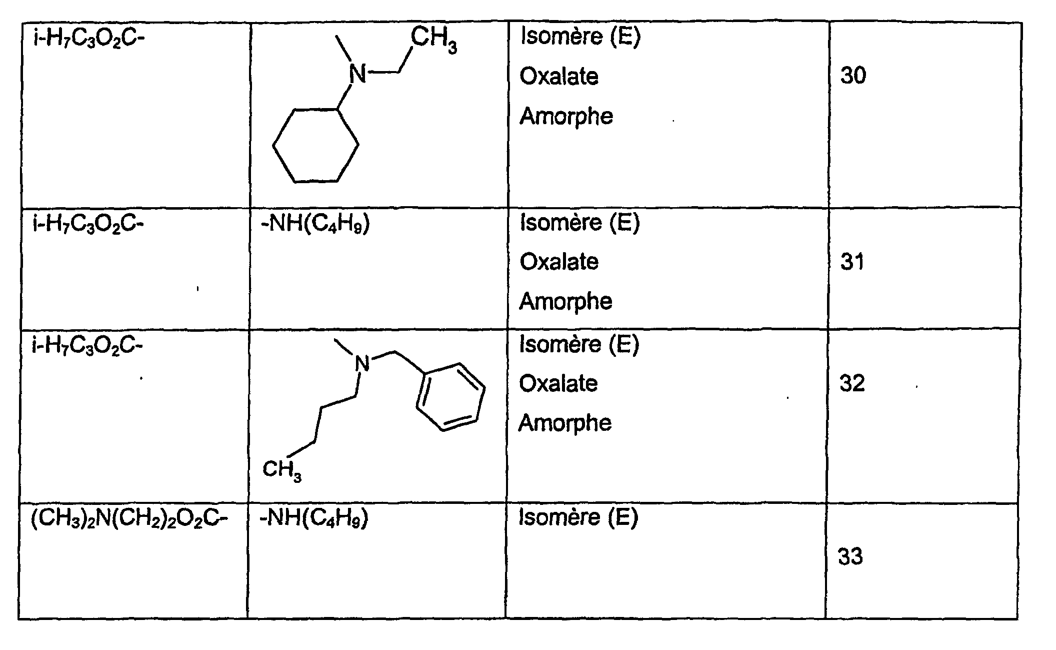

On a également préparé les composés listés ci-après.

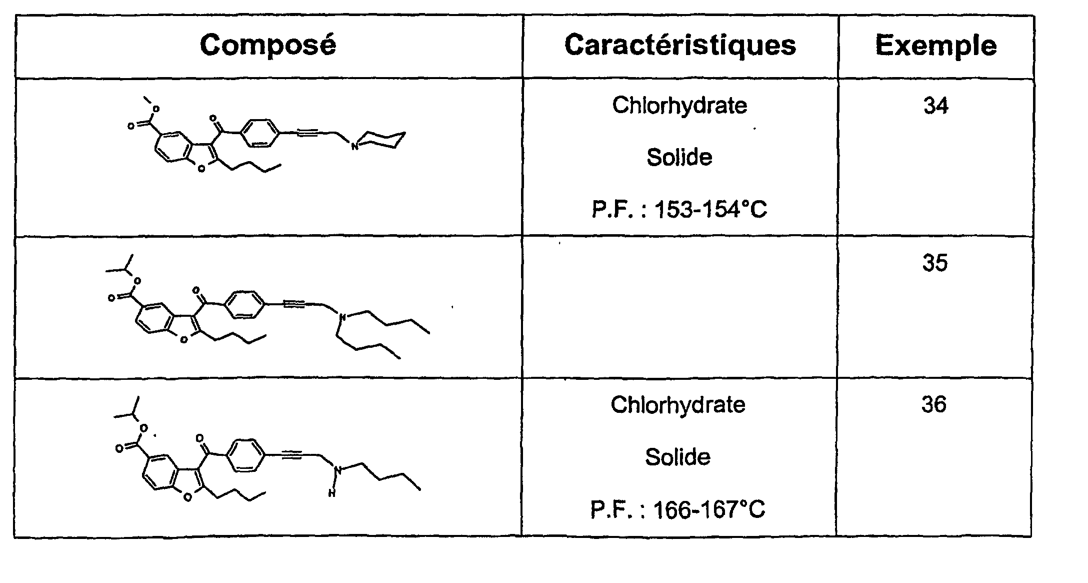

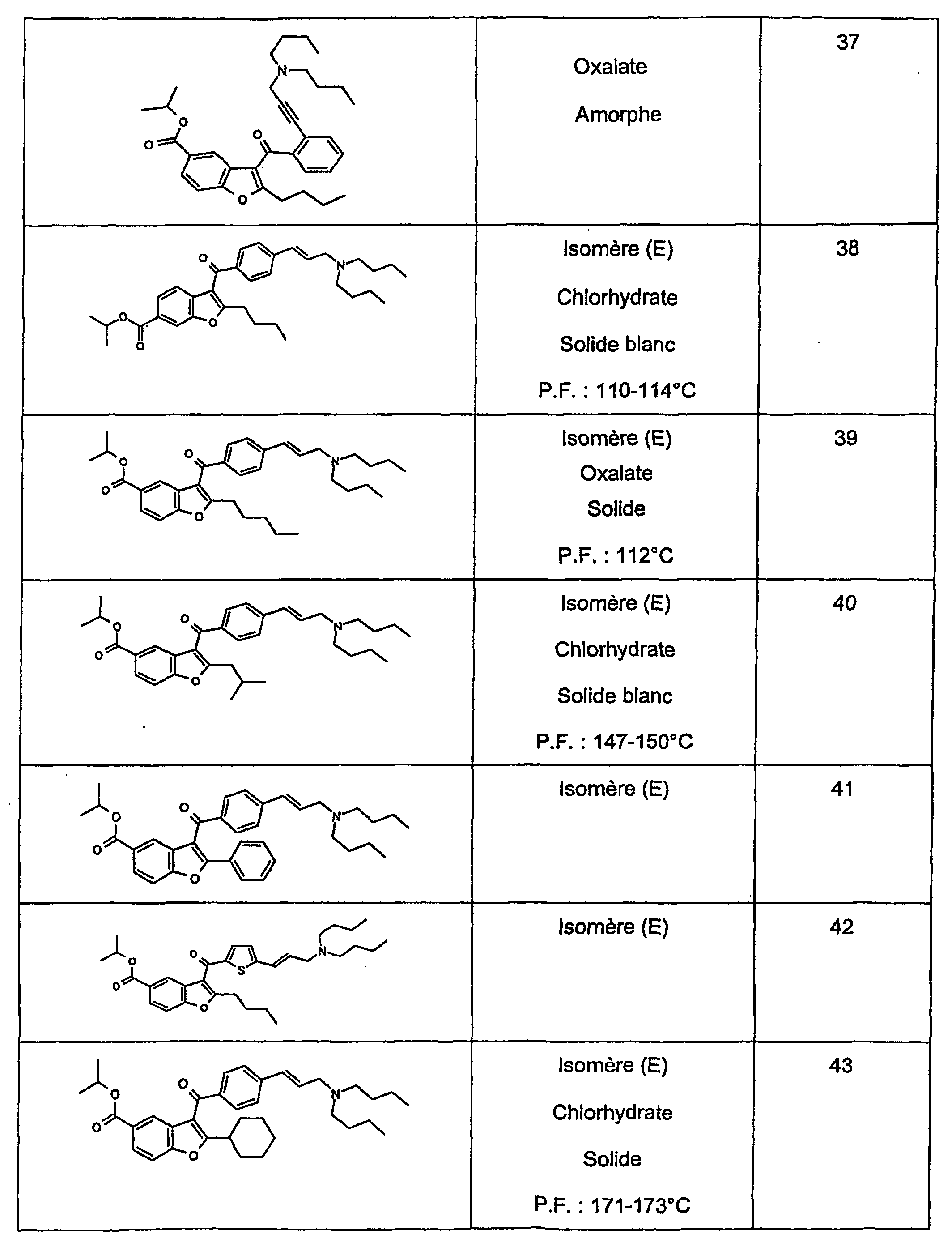

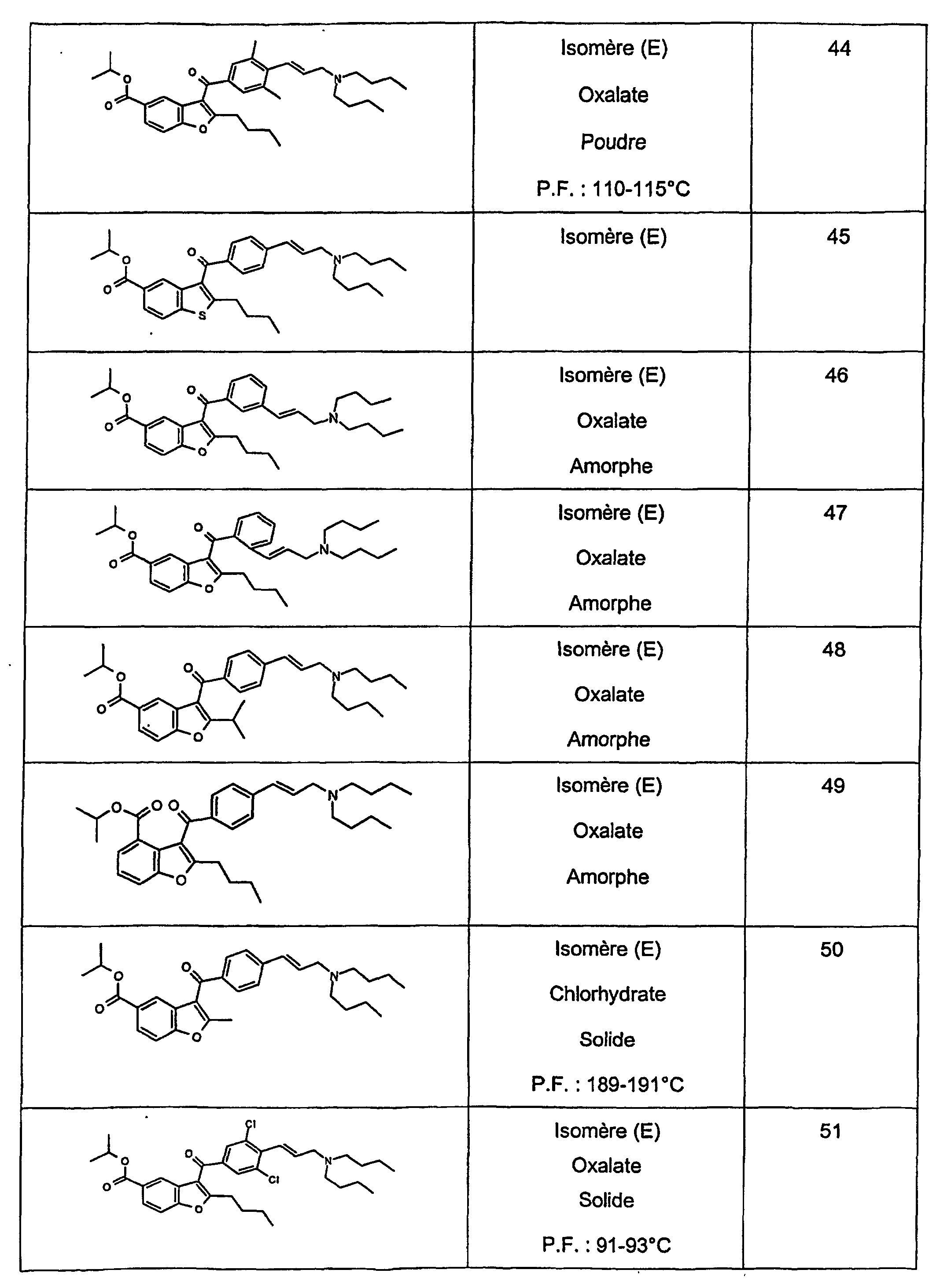

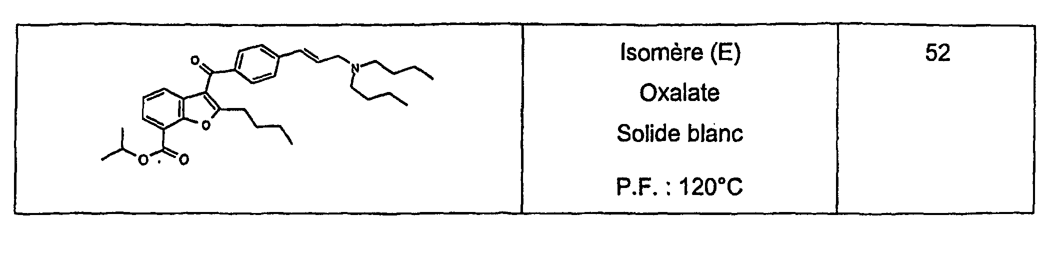

### Spectres R.M.N. à 200 MHz

### Exemple 12

Solvant: DMSO à 2,5 ppm
- δ (ppm) :: 0,85 ; triplet ; 3H, CH₃
1,1 à 2 ; massif ; 10H, 5CH₂
2,6 à 3 ; massif ; 6H, CH₂, 2NCH₂
3,85 ; singulet ; 3H, OCH₃
4,1 ; multiplet ; 2H, NCH₂
6,2 ; doublet de triplet ; 1 H, CH
7 ; doublet ; 1 H, CH
7,4 à 8,2 ; massif ; 7H, ¹H aromatique
10,8 ; singulet élargi ; 1H, NH⁺

### Exemple 13

Solvant: DMSO à 2,5 ppm
- δ (ppm) :: 0,75 ; triplet ; 3H, CH₃
0,9 à 2,1 ; massif; 15H, CH₃, 7CH₂
2,75 ; triplet; 2H, CH₂
2,8 à 3,5 ; massif ; 3H, NCH₂, NCH
3,8 ; singulet; 3H, OCH₃
4 ; multiplet ; 2H, NCH₂
6,15 ; doublet de triplet ; 1H, CH
6,95 ; doublet ; 1H, CH
7,35 à 8,15 ; massif ; 7H, ¹H aromatique
10,4 ; singulet élargi ; 1H, NH⁺

### Exemple 16

Solvant: DMSO à 2,5 ppm
- δ (ppm) :: 0,7 ; triplet; 3H, CH₃
1,0 à 2 ; massif ; 16H, 2CH₃, 5CH₂
2,55 à 3,5 ; massif ; 6H, CH₂, 2NCH₂
3,95 ; multiplet; 2H, CH₂N
5 ; septuplet ; 1H, OCH
6,05 ; doublet de triplet ; 1H, CH
6,9 ; doublet ; 1H, CH
7,3 à 8 ; massif; 7H, ¹H aromatique
10,6 ; singulet élargi ; 1H, NH⁺

### Exemple 17

Solvant : DMSO à 2,5 ppm
- δ (ppm) :: 0,7 ; triplet; 3H, CH₃
0,85 à 2,1 ; massif ; 23H, 3CH₃, 7CH₂
2,7 ; triplet; 2H, CH₂
2,8 à 3,3 ; massif ; 3H, NCH₂, NCH
4 ; multiplet; 2H, NCH₂
5,15 ; septuplet ; 1H, OCH
6,15 ; doublet de triplet ; 1H, CH
6,9 ; doublet ; 1H, CH
7,3 à 8 ; massif ; 7H, ¹H aromatique
10,7 ; singulet élargi ; 1H, NH⁺

### Exemple 30

Solvant : DMSO
δ (ppm) : 6.4-8.2 (massif, 9H) ; 5.09 (quintuplet, 1H) ; 3.0-4.2 (massif, 5H) ; 2.80 (triplet, 2H) ; 1.0-2.3 (massif, 23H) ; 0.78 (triplet, 3H)

### Exemple 37

Solvant : DMSO
δ (ppm) : 7.4-8.4 (massif, 7H) ; 5.12 (quintuplet, 1H) ; 3.69 (singulet, 2H) ; 2.4-2.7 (massif, 2H); 2.78 (triplet, 4H) ; 0.6-1.7 (massif, 27H)

### Exemple 46

Solvant : DMSO
δ (ppm) : 6.2-8.2 (massif, 9H) ; 5.09 (quintuplet, 1H) ; 3.69 (doublet large, 2H) ; 3.04 (doublet dédoublé large, 4H) : 2.76 (triplet, 2H) ; 0.6-1.9 (massif, 27H)

### Exemple 48

Solvant : DMSO
δ (ppm) : 6.5-8.0 (massif, 9H) ; 5.08 (quintuplet, 1H) ; 3.89 (doublet large, 2H) ; 3.21 (quintuplet, 1H) ; 3.03 (doublet dédoublé large, 4H) ; 1.2-1.8 (massif, 20H) ; 0.90 (triplet, 6H)

### Exemple 49

Solvant : DMSO
δ (ppm) : 6.3-8.0 (massif, 9H) ; 4.60 (quintuplet, 1H) ; 3,83 (doublet large, 2H) ; 2.96 (doublet dédoublé large, 4H) ; 2.73 (triplet, 2H) ; 0.6-1.8 (massif, 27H)

### EXEMPLE 22

Selon des techniques pharmaceutiques connues, on a préparé une capsule contenant les ingrédients suivants :

| **Ingrédient** | **mg** |
|---|---|
| Composé de l'invention | 100,0 |
| Amidon | 99,5 |
| Silice colloïdale | 0,5 |

## Revendications

1. Dérivés de benzofurane ou de benzothiophène de formule générale : 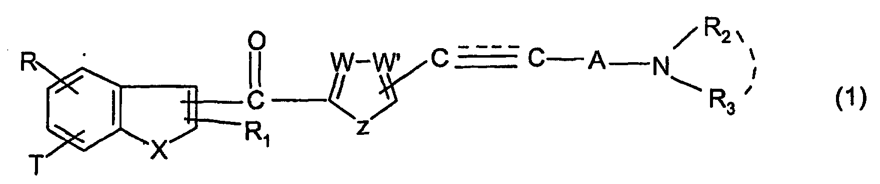 ainsi que leurs sels pharmaceutiquement acceptables, dans laquelle :
C 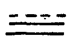 C représente le groupement -CH=CH- ou le groupement -C≡C-
A représente un groupement alkylène, linéaire ou ramifié, en C₁-C₃ ou alkénylène en C₂-C₃,
T représente l'hydrogène ou un radical alkyle en C₁-C₄,
R représente :
• le groupement cyano, hydroxyméthyle, formyle ou tétrazolyle,
• un groupement ester de formule générale: 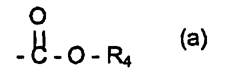 dans laquelle R₄ représente un groupement alkyle en C₁-C₆ ou cycloalkyle en C₃-C₆,
• un groupement carboxyle de formule générale: 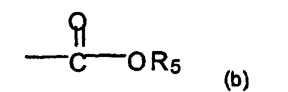 dans laquelle R₅ représente l'hydrogène ou un atome de métal alcalin,
• un groupement amide de formule générale : 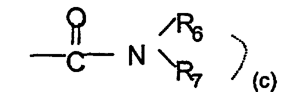 dans laquelle R₆ et R₇, identiques ou différents, représentent l'hydrogène ou un radical alkyle, linéaire ou ramifié, en C₁-C₄ ou R₆ et R₇ , lorsqu'ils sont pris ensemble, représentent une chaîne alkylène en C₂-C₆,
• un groupement de formule générale : 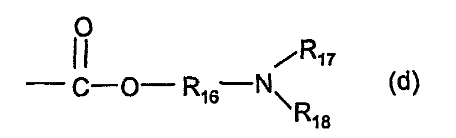 dans laquelle R₁₆ , R₁₇ et R₁₈ , identiques ou différents, représentent un groupement alkylène, linéaire ou ramifié, en C₁-C₄,
R₁ représente l'hydrogène, un groupement alkyle, linéaire ou ramifié, en C₁-C₆ , cycloalkyle en C₃-C₆, ou phényle,
R₂ et R₃, identiques ou différents, représentent l'hydrogène, un groupement alkyle, linéaire ou ramifié, en C₁-C₆ ou un groupement cycloalkyle en C₃-C₆,
ou R₂ et R₃, lorsqu'ils sont pris ensemble, représentent un groupement alkylène en C₃-C₁₀, linéaire ou ramifié,
W, W' et Z sont tels que :
- lorsque W et W', identiques représentent CH, Z représente -O- ou -S-
- lorsque W représente CH et W' représente C-R₈, Z représente 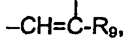 R₈ et R₉ étant identiques ou différents et représentant l'hydrogène, un atome d'halogène, un radical alkyle en C₁-C₄ ou un radical alkoxy en C₁-C₄,
X représente -O- ou -S-,
ces dérivés de benzofurane ou de benzothiphène étant sous formes d'isomères individuels ou de mélanges de ceux-ci.

2. Dérivés de benzofurane ou de benzothiophène selon la Revendication 1, **caractérisés en ce que**
C 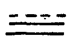 C représente le groupement -CH=CH-

3. Dérivés de benzofurane ou de benzothiophène selon la Revendication 1 ou 2, **caractérisés en ce que** R représente le groupement isopropoxycarbonyle.

4. Dérivés de benzofurane ou de benzothiophène selon une des Revendication à 3, **caractérisés en ce que** R₁ et/ou R₂ et/ou R₃ représentent le groupement n-butyle.

5. Dérivés de benzofurane ou de benzothiophène selon une des Revendications 1 à 4, **caractérisés en ce que** X représente - O -

6. Dérivés de benzofurane ou de benzothiophène selon une des Revendications 1 à 5, dans laquelle : 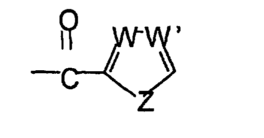 représente le groupement benzoyle

7. Dérivés de benzofurane ou de benzothiophène selon une des Revendications 1 à 6, dans laquelle : 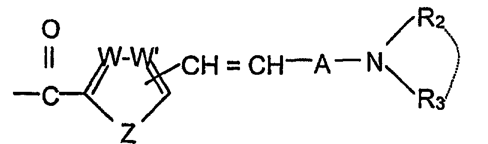 représente le groupement benzoyle substitué en position 4 par un groupement

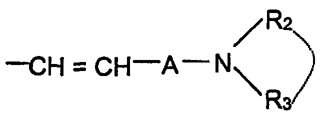

8. Dérivés de benzofurane ou de benzothiophène selon une des Revendications 1 à 7, **caractérisés en ce que** R₁ représente le groupement n-butyle, A représente le groupement méthylène et R₂ et R₃ identiques représentent le groupement n-butyle.

9. Dérivés de benzofurane ou de benzothiophène selon une des Revendications 1 à 8, **caractérisés en ce qu'**ils sont sous forme d'isomères de configuration E.

10. Dérivés de benzofurane ou de benzothiophène selon une des Revendications 1 à 8, **caractérisés en ce qu'**ils sont sous forme d'isomères de configuration Z.

11. 2-Butyl-3-[4-[(E)-3-(dibutylamino)-1-propenyl]benzoyl]-1-benzofurane-5-carboxylate d'isopropyle et ses sels pharmaceutiquement acceptables.

12. 2-Butyl-3-[4-[(Z)-3-(dibutylamino)-1-propenyl]benzoyl]-1-benzofurane-5-carboxylate d'isopropyle et ses sels pharmaceutiquement acceptables.

13. Dérivés de benzofurane ou de benzothiophène selon une des Revendications 1 à 12, dans lesquels le sel pharmaceutiquement acceptable est choisi parmi les maléate, fumarate, méthanesulfonate, benzoate, ascorbate, pamoate, succinate, hexamate, bisméthylènesalicylate, acétate, propionate, tartrate, salicylate, citrate, gluconate, lactate, malate, cinnamate, mandélate, citraconate, aspartate, palmitate, stéarate, itaconate, glycolate, p-aminobenzoate, glutamate, benzènesulfonate, p-toluènesulfonate et théophylline acétate ainsi que les sels formés à partir d'un acide aminé tel que le sel de lysine ou l'histidine.

14. Dérivés de benzofurane ou de benzothiophène selon une des Revendications 1 à 12, dans lesquels le sel pharmaceutiquement acceptable est choisi parmi les chlorhydrate, bromhydrate, sulfate, sulfamate, phosphate et nitrate.

15. Chlorhydrate de 2-butyl-3-[4-[(E)-3-(dibutylamino)-1-propenyl]benzoyl]-1-benzofurane-5-carboxylate d'isopropyle.

16. Procédé de préparation des dérivés de benzofurane ou de benzothiophène selon la Revendication 1 dans laquelle R représente le groupement cyano, le groupement formyle, un groupement (a) ou un groupement (c), ces dérivés étant sous forme d'isomères de configuration E, **caractérisés en ce que** l'on fait réagir un composé de formule générale: 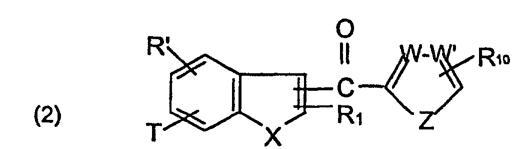 dans laquelle R' représente le groupement cyano, le groupement formyle, un groupement (a) ou un groupement (c), R₁₀ représente un atome d'halogène ou le groupement trifluorométhanesulfonyloxy et R₁, T, X, W, W' et Z ont la même signification que dans la Revendication 1, avec un dérivé organostannique de configuration E répondant à la formule générale : 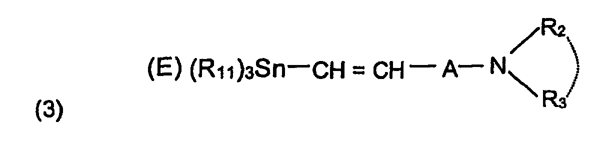 dans laquelle A, R₂ et R₃ ont la même signification que dans la Revendication 1 et R₁₁ représente un radical alkyle en C₁-C₄ après protection de la fonction amine lorsque R₂ et/ou R₃ représentent l'hydrogène et ce, en présence de chlorure de lithium et d'un dérivé organopalladié, puis on déprotège, si nécessaire, le composé ainsi formé, ce qui fournit les composés désirés sous forme de base libre que l'on peut faire réagir, si nécessaire, avec un acide organique ou inorganique pour former un sel pharmaceutiquement acceptable.

17. Procédé de préparation des dérivés de benzofurane ou de benzothiophène selon la Revendication 1 dans laquelle R représente le groupement cyano, le groupement formyle, un groupement (a) ou un groupement (c), ces dérivés étant sous forme d'isomères de configuration Z, **caractérisés en ce que** l'on hydrogène un composé alkynyle de formule générale : 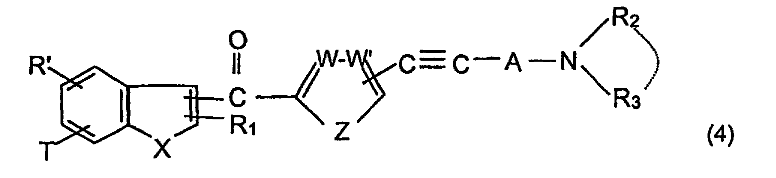 dans laquelle R' représente le groupement cyano, le groupement formyle, un groupement (a) ou un groupement (c) et A, R₁, R₂, R₃, T, W, W', X et Z ont la même signification que dans la Revendication 1 et ce, en présence d'un catalyseur approprié, ce qui fournit les composés désirés sous forme de base libre que l'on peut faire réagir, si nécessaire, avec un acide organique ou inorganique pour former un sel pharmaceutiquement acceptable.

18. Procédé de préparation de dérivés de benzofurane ou de benzothiophène selon la Revendication 1, dans laquelle R représente un groupement (b), **caractérisé en ce que** l'on saponifie en présence d'un hydroxyde de métal alcalin, un composé de formule générale : 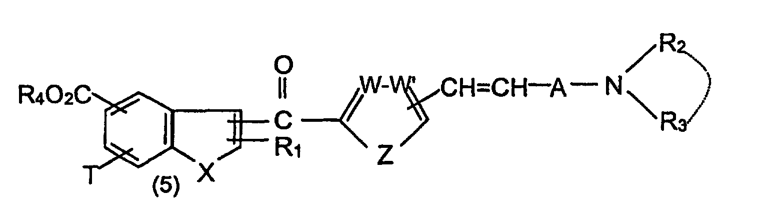 dans laquelle A, R₁, R₂, R₃, R₄, T, W, W' et Z ont la même signification que dans la Revendication 1, ce qui fournit, sous forme de base libre, les composés désirés de formule (1) dans laquelle R₅ représente un atome de métal alcalin, composés que l'on traite, si nécessaire, avec un acide fort, ce qui fournit sous forme de base libre, les composés désirés de formule (1) dans laquelle R₅ représente l'hydrogène, la base libre ainsi formée pouvant, si nécessaire, être traitée avec un acide organique ou inorganique approprié pour obtenir un sel pharmaceutiquement acceptable.

19. Procédé de préparation de dérivés de benzofurane ou de benzothiophène selon la Revendication 1, dans laquelle R représente le groupement hydroxyméthyle, **caractérisé en ce que** l'on déprotège un cétal de formule générale : 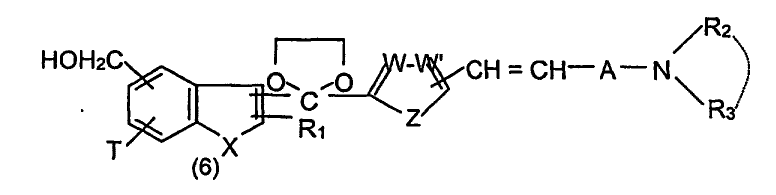 dans laquelle A, R₁, R₂, R₃, T, X, W, W' et Z ont la même signification que dans la Revendication 1 et ce, au moyen de pyridine p-toluènesulfonate, ce qui fournit les composés dérivés sous forme de base libre que l'on peut faire réagir, si nécessaire, avec un acide organique ou inorganique pour former un sel pharmaceutiquement acceptable.

20. Procédé de préparation de dérivés de benzofurane ou de benzothiophène selon la Revendication 1, dans laquelle R représente un groupement (c) dans lequel R₆ et R₇ sont identiques et représentent chacun l'hydrogène, **caractérisé en ce que** l'on fait réagir un composé de formule générale : 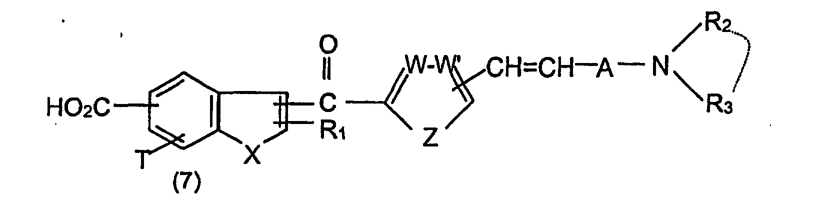 dans laquelle A, R₁, R₂, R₃, T, W, W, X et Z ont la même signification que dans la Revendication 1, au moyen de dicyclohexylcarbodiimide en présence d'hydroxybenzotriazole et d'ammoniaque, ce qui fournit les composés désirés sous forme de base libre que l'on peut faire réagir, si nécessaire, avec un acide organique ou inorganique pour former un sel pharmaceutiquement acceptable.

21. Procédé de préparation de dérivés de benzofurane ou de benzothiophène selon la Revendication 1 dans laquelle R représente un groupement (c) dans lequel R₆ et R₇ représentent chacun l'hydrogène, **caractérisé en ce que** l'on hydrolyse, en présence d'un acide fort, un composé de formule générale: 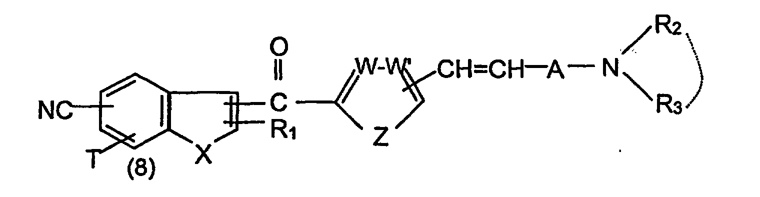 dans laquelle A, R₁, R₂, R₃, T, W, W', X et Z ont la même signification que dans la Revendication 1, ce qui fournit les composés désirés sous forme de base libre que l'on peut faire réagir, si nécessaire, avec un acide organique ou inorganique pour former un sel pharmaceutiquement acceptable.

22. Procédé de préparation de dérivés de benzofurane ou de benzothiophène selon la Revendication 1, dans laquelle R représente le groupement tétrazolyle, **caractérisé en ce que** l'on fait réagir, avec un (trialkyl en C₁-C₄) azido étain, un composé de formule générale : 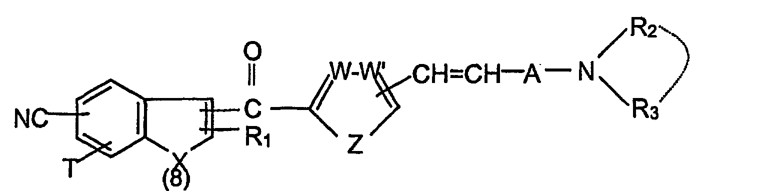 dans laquelle A, R₁, R₂, R₃, T, W, W', X et Z ont la même signification que dans le Revendication 1, ce qui fournit les composés désirés sous forme de base libre que l'on peut faire réagir, si nécessaire, avec un acide organique ou inorganique pour former un sel pharmaceutiquement acceptable.

23. Procédé de préparation de dérivés de benzofurane ou de benzothiophène selon la Revendication 16, **caractérisé en ce que** la protection de la fonction amine s'effectue au moyen d'un anhydride de t-butoxycarbonyle et la déprotection par traitement en milieu acide.

24. Médicament, **caractérisé en ce qu'**il comprend un dérivé de benzofurane ou de benzothiophène, ou un sel pharmaceutiquement acceptable de ce dernier, selon l'une quelconque des revendications 1 à 15.

25. Composition pharmaceutique ou vétérinaire, **caractérisée en ce qu'**elle contient comme principe actif au moins un dérivé de benzofurane ou de benzothiophène ou un sel pharmaceutiquement acceptable de ce dernier, selon une des Revendications 1 à 15, en association avec un véhicule pharmaceutique ou excipient approprié

26. Composition pharmaceutique ou vétérinaire selon la Revendication 25 pour le traitement de syndromes pathologiques du système cardio-vasculaire.

27. Composition pharmaceutique ou vétérinaire selon la Revendication 25 ou 26, pour le traitement de l'angine de poitrine, de l'hypertension, de l'arythmie atriale, ventriculaire ou supraventriculaire, de l'insuffisance circulatoire cérébrale, de l'insuffisance cardiaque, de l'infarctus du myocarde compliqué ou non d'insuffisance cardiaque ou pour la prévention de la mortalité post-infarctus.

28. Composition pharmaceutique ou vétérinaire selon une des Revendications 25 à 27, **caractérisée en ce qu'**elle contient de 50 à 500 mg de principe actif.

29. Utilisation d'au moins un dérivé de benzofurane ou de benzothiophène ou d'un sel pharmaceutiquement acceptable de ce dernier, selon une des Revendications 1 à 15, pour la fabrication d'un médicament destiné au traitement de syndromes pathologiques du système cardio-vasculaire.

## Patentansprüche

1. Benzofuran- oder Benzothiophen-Derivate der allgemeinen Formel: 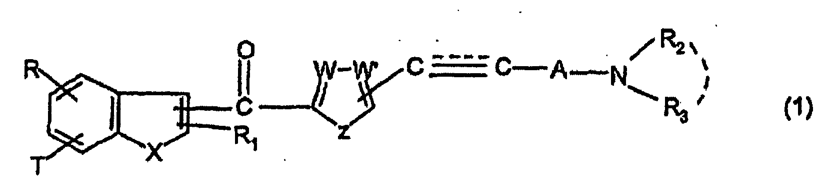 sowie deren pharmazeutisch annehmbare Salze, in der:
C 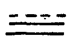 C die Gruppe -CH=CH- oder die Gruppe -C ≡C- bedeutet,
A eine geradkettige oder verzweigte C₁-C₃-Alkylen- oder C₂-C₃-Alkenylengruppe darstellt,
T Wasserstoff oder eine C₁-C₄-Alkylgruppe bedeutet, R:
• die Cyano-, Hydroxymethyl-, Formyl- oder Tetrazolylgruppe,
• eine Estergruppe der allgemeinen Formel: 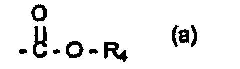 in der R₄ eine C₁-C₆-Alkylgruppe oder C₃-C₆-Cycloalkylgruppe darstellt.
• eine Carboxylgruppe der allgemeinen Formel: 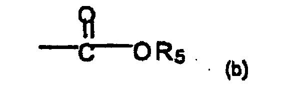 in der R₅ Wasserstoff oder ein Alkalimetallatom darstellt,
• eine Amidgruppe der allgemeinen Formel: 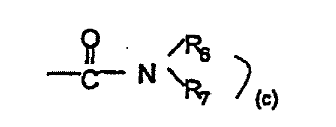 in der R₆ und R₇, die gleichartig oder verschieden sind, Wasserstoff oder geradkettige oder verzweigte C₁-C₄-Alkylgruppen bedeuten oder R₆ und R₇ gemeinsam eine C₂-C₆-Alkylenkette darstellen,
• eine Gruppe der allgemeinen Formel: 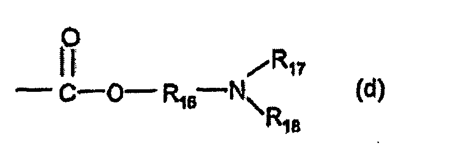 in der R₁₆, R₁₇ und R₁₈, die gleichartig oder verschieden sind, eine geradkettige oder erzweigte C₁-C₄-Alkylengruppe darstellen, bedeutet,
R₁ Wasserstoff, eine geradkettige oder verzweigte C₁-C₆-Alkylgruppe, C₃-C₆-Cycloalkylgruppe oder Phenylgruppe bedeutet,
R₂ und R₃, die gleichartig oder verschieden sind, Wasserstoff, eine geradkettige oder verzweigte C₁-C₆-Alkylgruppe oder eine C₃-C₆-Cycloalkylgruppe
oder R₂ und R₃ gemeinsam eine geradkettige oder verzweigte C₃-C₁₀-Alkylengruppe bedeuten,
W, W' und Z solche Bedeutungen besitzen, daß:
- wenn W und W' identisch sind und CH bedeuten, Z -O- oder -S- darstellt,
- wenn W CH und W' C-R₈ bedeuten, Z -CH=C-R₉ bedeutet, worin R₈ und R₉ gleichartig oder verschieden sind und Wasserstoff, ein Halogenatom, eine C₁-C₄-Alkylgruppe oder eine C₁-C₄-Alkoxygruppe darstellen,
X -O- oder -S- bedeutet,
wobei diese Benzofuran- oder Benzothiophen-Derivate in Form von getrennten Isomeren oder in Form von Mischungen davon vorliegen.

2. Benzofuran- oder Benzothiophen-Derivate nach Anspruch 1, **dadurch gekennzeichnet, daß** C 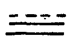 C die Gruppe -CH = CH- bedeutet.

3. Benzofuran- oder Benzothiophen-Derivate nach Anspruch 1 oder 2, **dadurch gekennzeichnet, daß** R eine Isopropoxycarbonylgruppe bedeutet.

4. Benzofuran- oder Benzothiophen-Derivate nach einem der Ansprüche 1 bis 3. **dadurch gekennzeichnet, daß** R₁ und/oder R₂ und/oder R₃ die n-Butylgruppe bedeuten.

5. Benzofuran- oder Benzothiophen-Derivate nach einem der Ansprüche 1 bis 4, **dadurch gekennzeichnet, daß** X -O- bedeutet.

6. Benzofuran- oder Benzothiophen-Derivate nach einem der Ansprüche 1 bis 5, worin 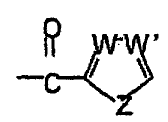 die Benzoylgruppe bedeutet.

7. Benzofuran- oder Benzothiophen-Derivate nach einem der Ansprüche 1 bis 6, worin 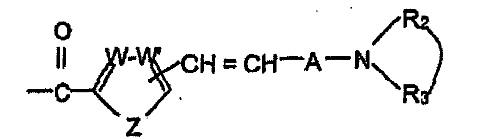 eine in der 4-Stellung durch eine Gruppe 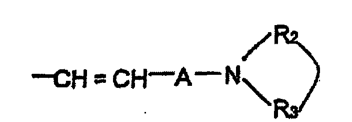 substituierte Benzoylgruppe bedeutet.

8. Benzofuran- oder Benzothiophen-Derivate nach einem der Ansprüche 1 bis 7, **dadurch gekennzeichnet, daß** R₁ die n-Butylgruppe, A die Methylengruppe und R₂ und R₃, die gleichartig sind, die n-Butylgruppe darstellen.

9. Benzofuran- oder Benzothiophen-Derivate nach einem der Ansprüche 1 bis 8, **dadurch gekennzeichnet, daß** sie in Form der Isomeren der Konfiguration E vorliegen.

10. Benzofuran- oder Benzothiophen-Derivate nach einem der Ansprüche 1 bis 8, **dadurch gekennzeichnet, daß** sie in Form der Isomeren der Konfiguration Z vorliegen.

11. 2-Butyl-3-[4-[(E)-3-(dibutylamino)-1-propenyl]-benzoyl]-1-benzofuran-5-carbonsäureisopropylester und dessen pharmazeutisch annehmbare Salze.

12. 2-Butyl-3-[4-[(Z)-3-(dibutylamino)-1-propenyl]-benzoyl]-1-benzofuran-5-carbonsäureisopropylester und dessen pharmazeutisch annehmbare Salze.

13. Benzofuran- oder Benzothiophen-Derivate nach einem der Ansprüche 1 bis 12, worin das pharmazeutisch annehmbare Salz ausgewählt ist aus dem Maleat, Fumarat, Methansulfonat, Benzoat, Ascorbat, Pamoat, Succinat, Hexamat, Bismethylensalicylat, Acetat, Propionat, Tartrat, Salicylat, Citrat, Gluconat, Lactat, Malat, Cinnamat, Mandelat, Citraconat, Aspartat, Palmitat, Stearat, Itaconat, Glykolat, p-Aminobenzoat, Glutamat, Benzolsulfonat, p-Toluolsulfonat und Theophyllinacetat sowie die Salze, die ausgehend von einer Aminosäure gebildet worden sind, wie das Salz von Lysin oder Histidin.

14. Benzofuran- oder Benzothiophen-Derivate nach einem der Ansprüche 1 bis 12, worin das pharmazeutisch annehmbare Salz ausgewählt ist aus dem Hydrochlorid, dem Hydrobromid, dem Sulfat, dem Sulfamat, dem Phosphat und dem Citrat.

15. 2-Butyl-3-[4-[(E)-3-(dibutylamino)-1-propenyl]-benzoyl]-1-benzofuran-5-carbonsäureisopropylester-Hydrochlorid.

16. Verfahren zur Herstellung der Benzofuran- oder Benzothiophen-Derivate nach Anspruch 1, worin R die Cyanogruppe, die Formylgruppe, eine Gruppe (a) oder eine Gruppe (c) darstellt, wobei diese Derivate in Form der Isomeren der Konfiguration E vorliegen, **dadurch gekennzeichnet, daß** man eine Verbindung der allgemeinen Formel: 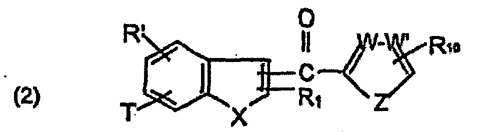 in der R' die Cyanogruppe, die Formylgruppe, eine Gruppe (a) oder eine Gruppe (c) darstellt, R₁₀ ein Halogenatom oder die Trifluormethansulfonyloxygruppe bedeutet und R₁, T, X, W, W' und Z die in Anspruch 1 angegebenen Bedeutungen besitzen, mit einem Zinn-organischen Derivat der Konfiguration E der allgemeinen Formel: 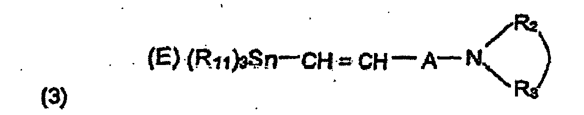 in der A, R₂ und R₃ die in Anspruch 1 angegebenen Bedeutungen besitzen und R₁₁ eine C₁-C₄-Alkylgruppe bedeutet, nach dem Schutz der Aminfunktion, wenn R₂ und/oder R₃ Wasserstoff bedeuten, in Gegenwart von Lithiumchlorid und eines Palladium-organischen Derivats umsetzt, dann erforderlichenfalls die in dieser Weise gebildete Verbindung von ihrer Schutzgruppe befreit, wodurch man die gewünschten Verbindungen in Form der freien Base erhält, welche man erforderlichenfalls mit einer anorganischen oder organischen Säure umsetzen kann zur Bildung eines pharmazeutisch annehmbaren Salzes.

17. Verfahren zur Herstellung der Benzofuran- oder Benzothiophen-Derivate nach Anspruch 1, worin R die Cyanogruppe, die Formylgruppe, eine Gruppe (a) oder eine Gruppe (c) darstellt, wobei diese Derivate in Form der Isomeren der Konfiguration Z vorliegen, **dadurch gekennzeichnet, daß** man eine Alkinylverbindung der allgemeinen Formel: 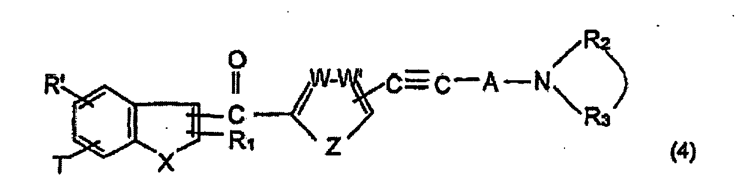 in der R' die Cyanogruppe, die Formylgruppe, eine Gruppe (a) oder eine Gruppe (c) darstellt und A, R₁, R₂, R₃, T, W, W', X und Z die in Anspruch 1 angegebenen Bedeutungen besitzen, in Gegenwart eines geeigneten Katalysators hydriert, so daß man die gewünschten Verbindungen in Form der freien Base erhält, die man erforderlichenfalls mit einer organischen oder anorganischen Säure zur Bildung eines pharmazeutisch annehmbaren Salzes umsetzen kann.

18. Verfahren zur Herstellung der Benzofuran- oder Benzothiophen-Derivate nach Anspruch 1, worin R eine Gruppe (b) darstellt, **dadurch gekennzeichnet, daß** man eine Verbindung der allgemeinen Formel: 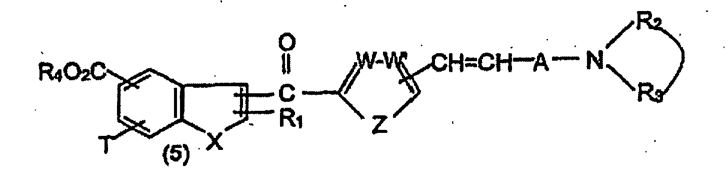 in der A, R₁, R₂, R₃, R₄, T, W, W' und Z die in Anspruch 1 angegebenen Bedeutungen besitzen, in Gegenwart eines Alkalimetallhydroxids verseift, so daß man die gewünschten Verbindungen der Formel (1), in der R₅ ein Alkalimetallatom bedeutet, in Form der freien Base erhält, welche Verbindungen man erforderlichenfalls mit einer starken Säure behandelt, so daß man die Verbindungen der Formel (1), in der R₅ Wasserstoff bedeutet, in Form der freien Base erhält, wobei man die in dieser Weise gebildete freie Base erforderlichenfalls mit einer organischen oder anorganischen Säure behandelt zur Bildung eines pharmazeutisch annehmbaren Salzes.

19. Verfahren zur Herstellung der Benzofuran- oder Benzothiophen-Derivate nach Anspruch 1, worin R die Hydroxymethylgruppe bedeutet, **dadurch gekennzeichnet, daß** man von einem Ketal der allgemeinen Formel: 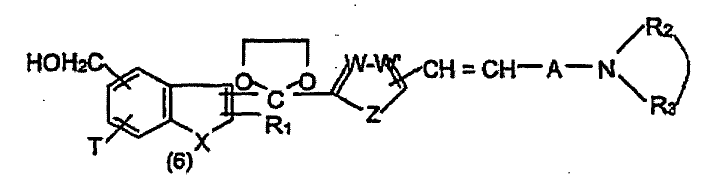 in der A, R₁, R₂, R₃, T, X, W, W' und Z die in Anspruch 1 angegebenen Bedeutungen besitzen, die Schutzgruppe mit Hilfe von Pyridin-p-toluolsulfonat abspaltet, so daß man die gewünschten Verbindungen in Form der freien Base erhält, die man erforderlichenfalls mit einer organischen oder anorganischen Säure umsetzen kann zur Bildung eines pharmazeutisch annehmbaren Salzes.

20. Verfahren zur Herstellung der Benzofuran- oder Benzothiophen-Derivate nach Anspruch 1, worin R eine Gruppe (c) darstellt, in der R₆ und R₇ identisch sind und jeweils Wasserstoff bedeuten, **dadurch gekennzeichnet, daß** man eine Verbindung der allgemeinen Formel: 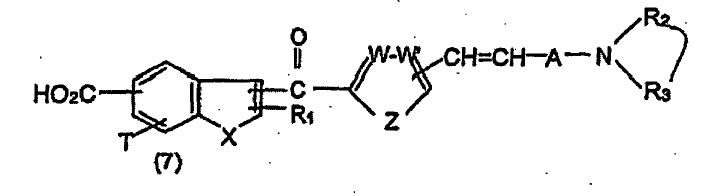 in der A, R₁, R₂, R₃, T, W, W', X und Z die in Anspruch 1 angegebenen Bedeutungen besitzen, mit Hilfe von Dicyclohexylcarbodiimid in Gegenwart von Hydroxybenzotriazol und Ammoniak umsetzt, wodurch man die gewünschten Verbindungen in Form der freien Base erhält, welche man erforderlichenfalls mit einer organischen oder anorganischen Säure umsetzen kann zur Bildung eines pharmazeutisch annehmbaren Salzes.

21. Verfahren zur Herstellung der Benzofuran- oder Benzothiophen-Derivate nach Anspruch 1, worin R eine Gruppe (c) bedeutet, in der R₆ und R₇ jeweils Wasserstoff darstellen, **dadurch gekennzeichnet, daß** man eine Verbindung der allgemeinen Formel: 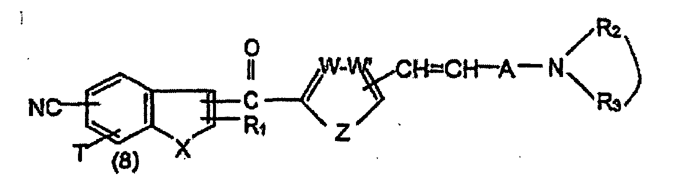 in der A, R₁, R₂, R₃, T, W, W', X und Z die in Anspruch 1 angegebenen Bedeutungen besitzen, in Gegenwart einer starken Säure hydrolysiert, wodurch man die gewünschten Verbindungen in Form der freien Base erhält, die man erforderlichenfalls mit einer organischen oder anorganischen Säure umsetzen kann zur Bildung eines pharmazeutisch annehmbaren Salzes.

22. Verfahren zur Herstellung der Benzofuran- oder Benzothiophen-Derivate nach Anspruch 1, worin R die Tetrazolylgruppe bedeutet, **dadurch gekennzeichnet, daß** man eine Verbindung der allgemeinen Formel: 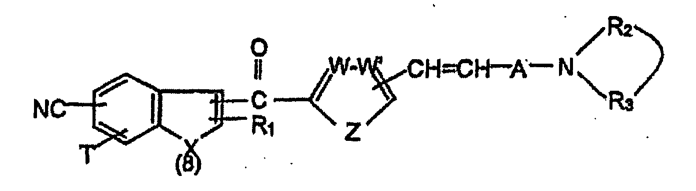 in der A, R₁, R₂, R₃, T, W, W', X und Z die in Anspruch 1 angegebenen Bedeutungen besitzen, mit einer (C₁-C₄-Trialkyl)-azido-zinnverbindung umsetzt, wodurch man die gewünschten Verbindungen in Form der freien Base erhält, die man erforderlichenfalls mit einer organischen oder anorganischen Säure umsetzen kann zur Bildung eines pharmazeutisch annehmbaren Salzes.

23. Verfahren zur Herstellung der Benzofuran- oder Benzothiophen-Derivate nach Anspruch 16, **dadurch gekennzeichnet, daß** man den Schutz der Aminfunktion mit Hilfe eines tert.-Butoxycarbonylanhydrids und die Abspaltung der Schutzgruppe durch Behandeln in saurem Medium bewirkt.

24. Arzneimittel, **dadurch gekennzeichnet, daß** es ein Benzofuran- oder Benzothiophen-Derivat oder ein pharmazeutisch annehmbares Salz davon nach einem der Ansprüche 1 bis 15 umfaßt.

25. Pharmazeutische oder veterinärmedizinische Zubereitung, **dadurch gekennzeichnet, daß** sie als Wirkstoff mindestens ein Benzofuran- oder Benzothiophen-Derivat oder ein pharmazeutisch annehmbares Salz davon nach einem der Ansprüche 1 bis 15 in Kombination mit einem geeigneten pharmazeutischen Trägermaterial oder Bindemittel enthält.

26. Pharmazeutische oder veterinärmedizinische Zubereitung nach Anspruch 25 zur Behandlung von pathologischen Syndromen des kardiovaskulären Systems.

27. Pharmazeutische oder veterinärmedizinische Zubereitung nach Anspruch 25 oder 26 zur Behandlung von Angina pectoris, der Hypertension, der atrialen, ventrikulären oder supraventrikulären Arrhythmie, der zerebralen Kreislaufinsuffizienz, der Herzinsuffizienz, des komplizierten oder insuffizienten Myokardinfarkts oder zur Vorbeugung der post-Infarktmortalität.

28. Pharmazeutische oder veterinärmedizinische Zubereitung nach einem der Ansprüche 25 bis 27, **dadurch gekennzeichnet, daß** sie 50 bis 500 mg des Wirkstoffs enthält.

29. Verwendung mindestens eines Benzofuran- oder Benzothiophen-Derivats oder eines pharmazeutisch annehmbaren Salzes davon nach einem der Ansprüche 1 bis 15 zur Herstellung eines Arzneimittels zur Behandlung von pathologischen Syndromen des kardiovaskulären Systems.

## Claims

1. Benzofuran or benzothiophene derivatives of general formula: 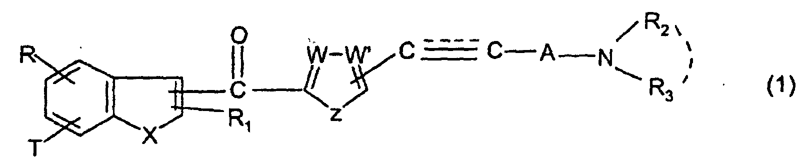 and their pharmaceutically acceptable salts, in which:
C 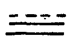 C represents the -CH=CH- group or the
-C≡C- group,
A represents a linear or branched C₁-C₃ alkylene group or a C₂-C₃ alkenylene group,
T represents hydrogen or a C₁-C₄ alkyl radical,
R represents:
• the cyano, hydroxymethyl, formyl or tetrazolyl group,
• an ester group of general formula: 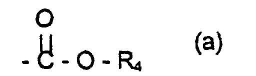 in which R₄ represents a C₁-C₆ alkyl or C₃-C₆ cycloalkyl group,
• a carboxyl group of general formula: 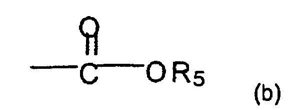
in which R₅ represents hydrogen or an alkali metal atom,
• an amide group of general formula: 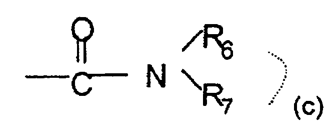 in which R₆ and R₇, which are identical or different, represent hydrogen or a linear or branched C₁-C₄ alkyl radical or R₆ and R₇, when they are taken together, represent a C₂-C₆ alkylene chain,
• a group of general formula: 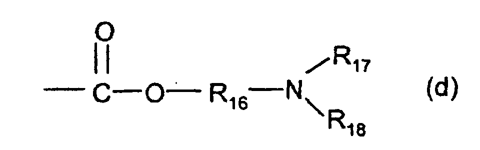
in which R₁₆, R₁₇ and R₁₈, which are identical or different, represent a linear or branched C₁-C₄ alkylene group,
R₁ represents hydrogen, a linear or branched C₁-C₆ alkyl group, a C₃-C₆ cycloalkyl group or a phenyl group,
R₂ and R₃, which are identical or different, represent hydrogen, a linear or branched C₁-C₆ alkyl group or a C₃-C₆ cycloalkyl group,
or R₂ and R₃, when they are taken together, represent a linear or branched C₃-C₁₀ alkylene group,
W, W' and Z are such that:
- when W and W', which are identical, represent CH, Z represents -O- or -S-
- when W represents CH and W' represents C-R₈, Z represents 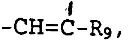 R₈ and R₉ being identical or different and representing hydrogen, a halogen atom, a C₁-C₄ alkyl radical, or a C₁-C₄ alkoxy radical,
X represents -O- or -S-,
these benzofuran or benzothiphene derivatives being in the form of individual isomers or of mixtures of the latter.

2. Benzofuran or benzothiophene derivatives according to Claim 1, **characterized in that** C 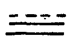 C represents the -CH=CH- group.

3. Benzofuran or benzothiophene derivatives according to Claim 1 or 2, **characterized in that** R represents the isopropoxycarbonyl group.

4. Benzofuran or benzothiophene derivatives according to one of Claims 1 to 3, **characterized in that** R₁ and/or R₂ and/or R₃ represent the n-butyl group.

5. Benzofuran or benzothiophene derivatives according to one of Claims 1 to 4, **characterized in that** X represents -O-.

6. Benzofuran or benzothiophene derivatives according to one of Claims 1 to 5, in which: 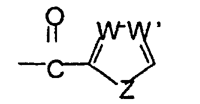 represents the benzoyl group.

7. Benzofuran or benzothiophene derivatives according to one of Claims 1 to 6, in which: 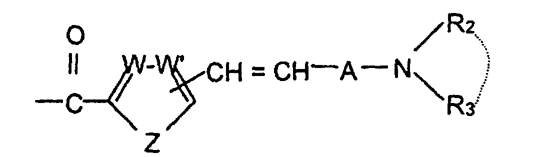 represents the benzoyl group substituted in the 4-position by a 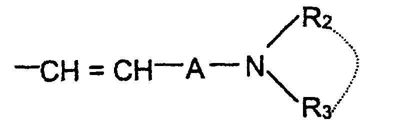 group.

8. Benzofuran or benzothiophene derivatives according to one of Claims 1 to 7, **characterized in that** R₁ represents the n-butyl group, A represents the methylene group and R₂ and R₃, which are identical, represent the n-butyl group.

9. Benzofuran or benzothiophene derivatives according to one of Claims 1 to 8, **characterized in that** they are in the form of isomers with the E configuration.

10. Benzofuran or benzothiophene derivatives according to one of Claims 1 to 8, **characterized in that** they are in the form of isomers with the Z configuration.

11. Isopropyl 2-butyl-3-[4-[(E)-3-(dibutylamino)-1-propenyl]benzoyl]-1-benzofuran-5-carboxylate and its pharmaceutically acceptable salts.

12. Isopropyl 2-butyl-3-[4-[(Z)-3-(dibutylamino)-1-propenyl]benzoyl]-1-benzofuran-5-carboxylate and its pharmaceutically acceptable salts.

13. Benzofuran or benzothiophene derivatives according to one of Claims 1 to 12, in which the pharmaceutically acceptable salt is chosen from the maleate, fumarate, methanesulphonate, benzoate, ascorbate, pamoate, succinate, hexamate, bismethylenesalicylate, acetate, propionate, tartrate, salicylate, citrate, gluconate, lactate, malate, cinnamate, mandelate, citraconate, aspartate, palmitate, stearate, itaconate, glycolate, p-aminobenzoate, glutamate, benzenesulphonate, p-toluenesulphonate and theophyllineacetate, and the salts formed from an amino acid, such as the lysine or histidine salt.

14. Benzofuran or benzothiophene derivatives according to one of Claims 1 to 12, in which the pharmaceutically acceptable salt is chosen from the hydrochloride, hydrobromide, sulphate, sulphamate, phosphate and nitrate.

15. Isopropyl 2-butyl-3-[4-[(E)-3-(dibutylamino)-1-propenyl]benzoyl]-1-benzofuran-5-carboxylate hydrochloride.

16. Process for the preparation of the benzofuran or benzothiophene derivatives according to Claim 1 in which R represents the cyano group, the formyl group, a group (a) or a group (c), these derivatives being in the form of isomers with the E configuration, **characterized in that** a compound of general formula: 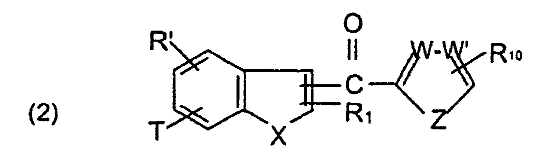 in which R' represents the cyano group, the formyl group, a group (a) or a group (c), R₁₀ represents a halogen atom or the trifluoromethanesulphonyloxy group and R₁, T, X, W, W' and Z have the same meaning as Claim 1, is reacted with an organotin derivative with the E configuration corresponding to the general formula: 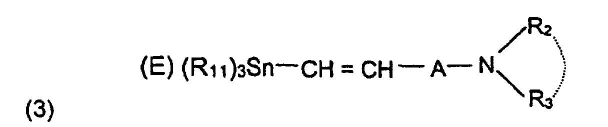 in which A, R₂ and R₃ have the same meaning as in Claim 1 and R₁₁ represents a C₁-C₄ alkyl radical, after protection of the amine functional group when R₂ and/or R₃ represent hydrogen, this reaction being carried out in the presence of lithium chloride and of an organopalladium derivative, and then, if necessary, the compound thus formed is deprotected, which gives the desired compounds in the free base form, which can be reacted, if necessary, with an organic or inorganic acid to form a pharmaceutically acceptable salt.

17. Process for the preparation of the benzofuran or benzothiophene derivatives according to Claim 1 in which R represents the cyano group, the formyl group, a group (a) or a group (c), these derivatives being in the form of isomers with the Z configuration, **characterized in that** an alkynyl compound of general formula: 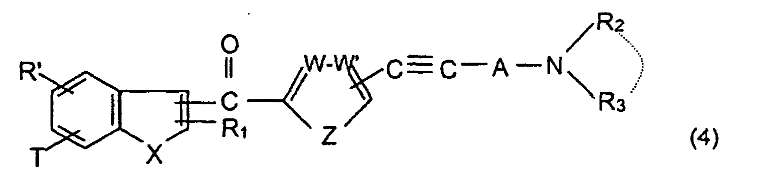 in which R' represents the cyano group, the formyl group, a group (a) or a group (c) and A, R₁, R₂, R₃, T, W, W', X and Z have the same meaning as in Claim 1, is hydrogenated, this reaction being carried out in the presence of an appropriate catalyst, which gives the desired compounds in the free base form, which can be reacted, if necessary, with an organic or inorganic acid to form a pharmaceutically acceptable salt.

18. Process for the preparation of benzofuran or benzothiophene derivatives according to Claim 1 in which R represents a group (b), **characterized in that** a compound of general formula: 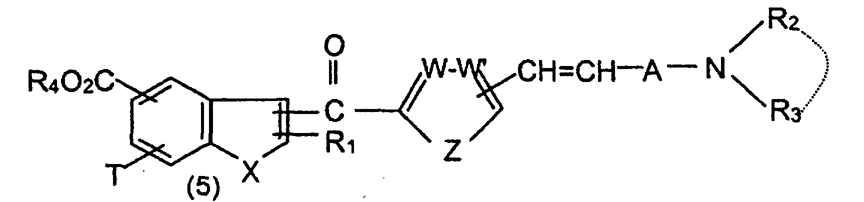 in which A, R₁, R₂, R₃, R₄, T, W, W' and Z have the same meaning as in Claim 1, is saponified in the presence of an alkali metal hydroxide, which gives, in the free base form, the desired compounds of formula (1) in which R₅ represents an alkali metal atom, which compounds are treated, if necessary, with a strong acid, which gives, in the free base form, the desired compounds of formula (1) in which R₅ represents hydrogen, it being possible for the free base thus formed to be treated, if necessary, with an appropriate organic or inorganic acid to obtain a pharmaceutically acceptable salt.

19. Process for the preparation of benzofuran or benzothiophene derivatives according to Claim 1 in which R represents the hydroxymethyl group, **characterized in that** a ketal of general formula: 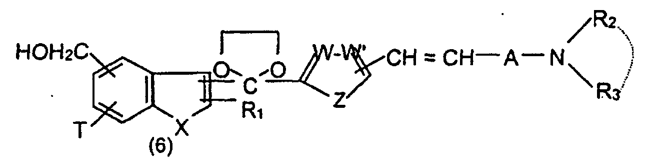 in which A, R₁, R₂, R₃, T, X, W, W' and Z have the same meaning as in Claim 1, is deprotected, this reaction being carried out by means of pyridine p-toluenesulphonate, which gives the derived compounds in the free base form, which can be reacted, if necessary, with an organic or inorganic acid to form a pharmaceutically acceptable salt.

20. Process for the preparation of benzofuran or benzothiophene derivatives according to Claim 1 in which R represents a group (c) in which R₆ and R₇ are identical and each represent hydrogen, **characterized in that** a compound of general formula: 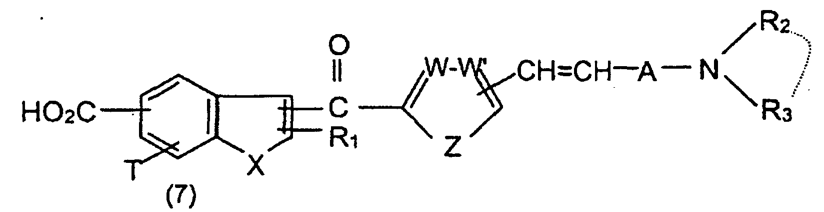 in which A, R₁, R₂, R₃, T, W, W', X and Z have the same meaning as in Claim 1, is reacted by means of dicyclohexylcarbodiimide in the presence of hydroxybenzotriazole and of ammonia, which gives the desired compounds in the free base form, which can be reacted, if necessary, with an organic or inorganic acid to form a pharmaceutically acceptable salt.

21. Process for the preparation of benzofuran or benzothiophene derivatives according to Claim 1 in which R represents a group (c) in which R₆ and R₇ each represent hydrogen, **characterized in that** a compound of general formula: 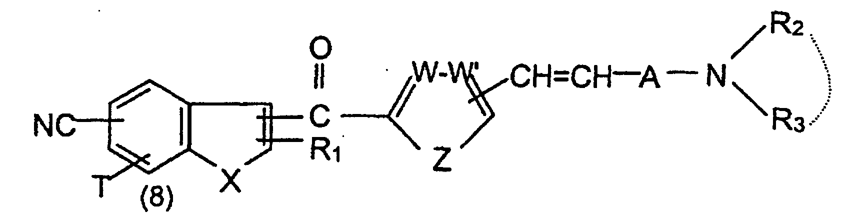 in which A, R₁, R₂, R₃, T, W, W', X and Z have the same meaning as in Claim 1, is hydrolysed in the presence of a strong acid, which gives the desired compounds in the free base form, which can be reacted, if necessary, with an organic or inorganic acid to form a pharmaceutically acceptable salt.

22. Process for the preparation of benzofuran or benzothiophene derivatives according to Claim 1 in which R represents the tetrazolyl group, **characterized in that** a compound of general formula: 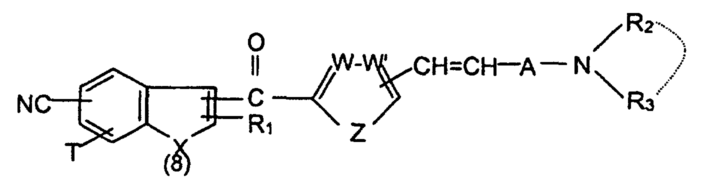 in which A, R₁, R₂, R₃, T, W, W', X and Z have the same meaning as in Claim 1, is reacted with a [tri(C₁-C₄ alkyl)]azidotin, which gives the desired compounds in the free base form, which can be reacted, if necessary, with an organic or inorganic acid to form a pharmaceutically acceptable salt.

23. Process for the preparation of benzofuran or benzothiophene derivatives according to Claim 16,
**characterized in that** the protection of the amine functional group is carried out by means of a t-butoxycarbonyl anhydride and the deprotection by treatment in an acidic medium.

24. Medicament, **characterized in that** it comprises a benzofuran or benzothiophene derivative, or a pharmaceutically acceptable salt of the latter, according to any one of Claims 1 to 15.

25. Pharmaceutical or veterinary composition, **characterized in that** it comprises, as active principle, at least one benzofuran or benzothiophene derivative, or a pharmaceutically acceptable salt of the latter, according to one of Claims 1 to 15, in combination with an appropriate excipient or pharmaceutical vehicle.

26. Pharmaceutical or veterinary composition according to Claim 25, for the treatment of pathological syndromes of the cardiovascular system.

27. Pharmaceutical or veterinary composition according to Claim 25 or 26, for the treatment of angina pectoris, hypertension, atrial, ventricular or supraventricular arrhythmia, cerebral circulatory insufficiency, cardiac insufficiency or myocardial infarction, complicated or not complicated by cardiac insufficiency, or for the prevention of postinfarction mortality.

28. Pharmaceutical or veterinary composition according to one of Claims 25 to 27, **characterized in that** it comprises from 50 to 500 mg of active principle.

29. Use of at least one benzofuran or benzothiophene derivative, or of a pharmaceutically acceptable salt of the latter, according to one of Claims 1 to 15, for the manufacture of a medicament intended for the treatment of pathological syndromes of the cardiovascular system.
